(19) Europäisches Patentamt
European Patent Office
Office européen des brevets

(11) EP 3 743 064 B1

(12) EUROPEAN PATENT SPECIFICATION

(45) Date of publication and mention of the grant of the patent:
**11.08.2021 Bulletin 2021/32**

(21) Application number: **19734023.5**

(22) Date of filing: **24.06.2019**

(51) Int Cl.:
***A61K 31/402*** (2006.01) ***A61P 13/12*** (2006.01)

(86) International application number:
**PCT/EP2019/066578**

(87) International publication number:
**WO 2019/243625 (26.12.2019 Gazette 2019/52)**

# (54) TREATMENT OF PROTEINURIA

BEHANDLUNG VON PROTEINURIE

TRAITEMENT DE LA PROTÉINURIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2018 EP 18179319**

(43) Date of publication of application:
**02.12.2020 Bulletin 2020/49**

(73) Proprietor: **SynAct Pharma ApS**
**2840 Holte (DK)**

(72) Inventor: **JONASSEN, Thomas Engelbrecht Nordkild**
**2840 Holte (DK)**

(74) Representative: **Høiberg P/S**
**Adelgade 12**
**1304 Copenhagen K (DK)**

(56) References cited:
**WO-A1-2007/141343**

- **ANNIKA LINDSKOG JONSSON ET AL: "Effects of Melanocortin 1 Receptor Agonists in Experimental Nephropathies", PLOS ONE, vol. 9, no. 1, 1 January 2014 (2014-01-01) , page e87816, XP055621490, DOI: 10.1371/journal.pone.0087816**
- **JOHANNES ELVIN ET AL: "Melanocortin 1 receptor agonist protects podocytes through catalase and RhoA activation", AMERICAN JOURNAL OF PHYSIOLOGY: RENAL PHYSIOLOGY, vol. 310, no. 9, 1 May 2016 (2016-05-01), pages F846-F856, XP055621166, United States ISSN: 1931-857X, DOI: 10.1152/ajprenal.00231.2015**
- **RUJUN GONG: "Leveraging Melanocortin Pathways to Treat Glomerular Diseases", ADVANCES IN CHRONIC KIDNEY DISEASE, vol. 21, no. 2, 1 March 2014 (2014-03-01), pages 134-151, XP055600424, USA ISSN: 1548-5595, DOI: 10.1053/j.ackd.2013.09.004**
- **A. LINDSKOG ET AL: "Melanocortin 1 Receptor Agonists Reduce Proteinuria", JOURNAL OF THE AMERICAN SOCIETY OF NEPHROLOGY., vol. 21, no. 8, 27 May 2010 (2010-05-27), pages 1290-1298, XP055349064, US ISSN: 1046-6673, DOI: 10.1681/ASN.2009101025 cited in the application**
- **YINGJIN QIAO ET AL: "MC1R is dispensable for the proteinuria reducing and glomerular protective effect of melanocortin therapy", SCIENTIFIC REPORTS, vol. 6, no. 1, 1 September 2016 (2016-09-01), XP055621155, DOI: 10.1038/srep27589 cited in the application**
- **TRINIDAD MONTERO-MELENDEZ ET AL: "Biased Agonism as a Novel Strategy To Harness the Proresolving Properties of Melanocortin Receptors without Eliciting Melanogenic Effects", THE JOURNAL OF IMMUNOLOGY, vol. 194, no. 7, 1 April 2015 (2015-04-01) , pages 3381-3388, XP055621293, US ISSN: 0022-1767, DOI: 10.4049/jimmunol.1402645**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 743 064 B1

## Description

### Technical field

The present disclosure relates to a composition comprising a compound of formula (I), (Ia) or (II), or a pharmaceutically acceptable derivative thereof, for use in a method of treating kidney disease, especially proteinuric kidney diseases, such as nephrotic syndromes.

### Background

Proteinuria, the hallmark of glomerular injury, is a common finding on urinalysis, and is by itself a strong, independent and modifiable risk factor for end stage renal disease, premature death of cardiovascular origin, and ischemic stroke in patients with diabetes. Despite recent advances refractory proteinuria continues to be a challenge in clinical practice. It is imperative to develop more effective modalities to ameliorate glomerular injury and induce remission of proteinuria. Recent evidence points to melanocortin system as a novel target for treatment of proteinuria.

Membranous nephropathy (MN) is one of the most common causes of nephrotic syndrome in adults. One-third of the patients have a good prognosis with spontaneous remission. Even so, approximately 50% of the remainder manifest an unchanged disease state, whereas 50% progress into ESRD (end-stage renal disease) and dialysis. The nephrotic syndrome is a glomerular disease characterized by proteinuria, edema, hypoalbuminemia, and hyperlipidemia. Most MN cases are idiopathic and the mechanisms underlying the disease are still largely unknown. Treatments have classically been nonspecific and often include steroids with anti-inflammatory actions, sometimes in combination with cytotoxic agents. These drugs affect multiple tissues, causing cytotoxic effects, osteoporosis, adrenal insufficiency, hypertension, peptic ulcers, and increased risk of glucose intolerance and infections. Therefore, more effective and specific treatment options are needed.

The melanocortin system is a set of neuropeptidergic and immunoendocrine signaling pathways that play an integral role in the homeostatic control of a diverse array of physiological functions, including melanogenesis, stress response, inflammation, immunomodulation and adrenocortical steroidogenesis. It consists of multiple components, including the five G protein-coupled melanocortin receptors: melanocortin receptor 1 (MC1R) to MC5R; peptide ligands: $\alpha$, $\beta$, $\gamma$-melanocyte stimulating hormone ($\alpha$, $\beta$, $\gamma$- MSH), adrenocorticotropic hormone (ACTH) secreted by the anterior pituitary; and endogenous antagonists. The biological functions of melanocortin system are mediated by the five melanocortin receptors (MCRs), which have distinct tissue distribution, convey different signalling and exert varying biological activities in different organ systems.

Adrenocorticotropic hormone (ACTH) is an endogenous peptide hormone and agonist for all melanocortin receptors 1 to 5 (MC1-5R), of which MC2R specifically binds ACTH; steroidogenesis is triggered only by ACTH and mediated via MC2R in the adrenal cortex. Alpha-melanocyte stimulating hormone ($\alpha$MSH) is a small endogenous peptide hormone, structurally related to ACTH, which binds all of the MCRs except MC2R. MC1R, abundantly expressed by melanocytes in the skin, is a key control point in melanogenesis and determines hair colour.

Melanocortin therapy by using ACTH or non-steroidogenic melanocortin peptides attenuates proteinuria and glomerular injury in experimental glomerular diseases and induces remission of nephrotic syndrome in patients with diverse glomerulopathies, even those resistant to steroids, including membranous nephropathy (MN), minimal change disease (MCD), focal segmental glomerulosclerosis (FSGS) and IgA nephropathy. For example, Lindskog-Jonsson et al. 2014, Elvin et al. 2016 and Gong et al. 2014 disclose melanocortin agonists for use in treating kidney disorders. The underlying mechanism remains elusive, but the role of melanocortin 1 receptor (MC1R) has been implicated and MC1R is heavily and specifically expressed in human kidney cortex and specific renal cell types including podocytes (Lindskog et al. 2010). However, recent studies have found effects of ACTH monotherapy and NDP-aMSH ([$Nle_4$, D-$Phe_7$]-$\alpha$MSH) also in patients bearing dominant-negative MC1R mutations, which was confirmed also in MC1R-null mice and in vitro in primary podocytes derived from MC1R-null and wildtype mice. The anti-proteinuric effect of melanocortin signalling thus appears to be steroidogenic-independent and MC1R-dispensable, and the melanocortin effect might, at least in part, target a pathogenic pathway common to all proteinuric kidney diseases. Podocytes are a critical component of the glomerular filtration barrier controlling glomerular permeability and selectivity and are considered a major culprit accounting for massive proteinuria in diverse glomerular diseases. The beneficial effect of melanocortin therapy is likely attributable to a direct action on podocytes (Qiao et al. 2016).

It is at present therefore not possible to predict which melanocortin agonists and which associated targets will present as useful candidates for treatment of proteinuric diseases such as kidney diseases.

Phenyl pyrrole aminoguanidine derivatives with activity on the melanocortin receptors are disclosed in WO 2007/141343. One example of such compound is the anti-inflammatory AP1189 (*(E)-N*-trans-{3-[1-(2-nitrophenyl)-1H-pyrrol-2-yl]-allylidene}-aminoguanidium acetate) which was first shown to bind the MC1R (WO 2007/141343) and later was identified as a biased dual agonist at receptors MC1R and MC3R that does not provoke canonical cAMP generation

(and hence no MC1R-induced melanogenesis) but instead appear to induce alternative pathways including ERK1/2-phosphorylation and $Ca^{2+}$ mobilization (Montero-Melendez et al. 2015).

**Summary**

The present inventors have found that the phenyl pyrrole aminoguanidine derivative AP1189 (*(E)*-*N*-trans-{3-[1-(2-nitrophenyl)-1H-pyrrol-2-yl]-allylidene}-aminoguanidium acetate) significantly reduces proteinuria in Passive Haymann Nephritis, a nephropathy model, as compared to vehicle, measured as a significant reduction of albumin excretion rate and $FR_{Alb}$ (fraction albumin excretion); and also has a significantly higher GFR (glomerular filtration rate; creatinine clearance) and a significantly reduced $FR_{Alb}$ compared to treatment with ACTH(1-24). This implies that AP1189 and related compounds are candidates for the improved treatment of proteinuric kidney diseases.

It is an aspect of the present disclosure to provide a composition comprising a compound of formula (I) or (Ia):

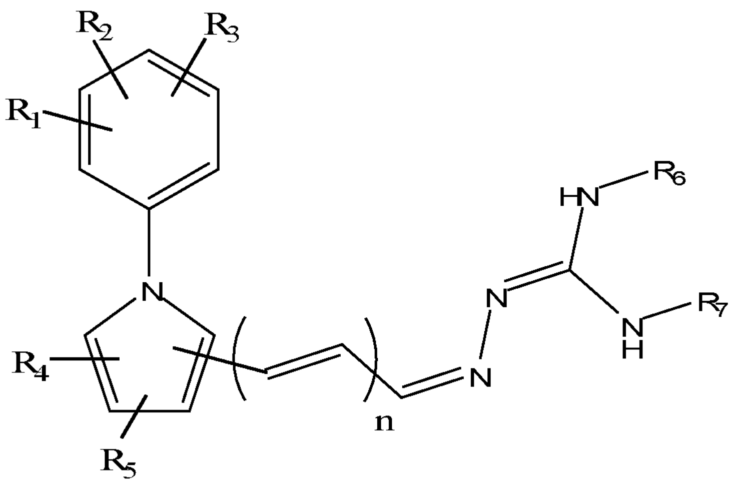

formula (I)

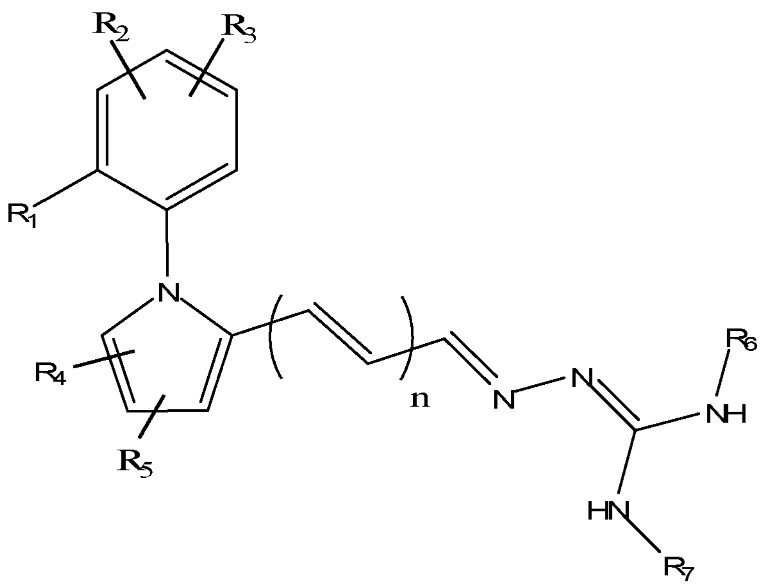

formula (Ia)

including tautomeric and isomeric forms, such as enantiomeric forms, diastereomeric forms and racemic forms, thereof; wherein

n is 1, 2 or 3;

each $R_1$, $R_2$, $R_3$, $R_4$ and $R_5$ is independently selected from the group consisting of hydrogen, optionally substituted $C_{1-6}$-alkyl, optionally substituted $C_{3-6}$-cycloalkyl,

optionally substituted $C_{2-6}$-alkenyl, optionally substituted $C_{4-6}$-alkadienyl, optionally substituted $C_{2-6}$- alkynyl, hydroxy, optionally substituted $C_{1-6}$-alkoxy, optionally substituted $C_{2-6}$-alkenyloxy, carboxy, optionally substituted $C_{1-6}$-alkoxycarbonyl, optionally substituted $C_{1-6}$- alkylcarbonyl, formyl, $C_{1-6}$-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, mono- and di($C_{1-6}$-alkyl)amino, carbamoyl, mono- and di($C_{1-6}$-alkyl)aminocarbonyl, amino-$C_{1-6}$-alkyl-aminocarbonyl, mono- and di($C_{1-6}$-alkylamino-$C_{1-6}$-alkyl-aminocarbonyl, $C_{1-6}$-alkylcarbonylamino, amino-$C_{1-6}$-alkyl- carbonylamino, mono- and di($C_{1-6}$-alkyl)amino-$C_{1}$-$_{6}$-alkyl-carbonylamino, cyano, guanidino, carbamido, $C_{1-6}$-alkanoyloxy, $C_{1-6}$-alkylsulphonyl, $C_{1-6}$-alkylsulphinyl, $C_{1-6}$-alkylsulphonyl- oxy, aminosulfonyl, mono- and di($C_{1-6}$-alkyl)aminosulfonyl, nitro, optionally substituted $C_{1-6}$-alkylthio and halogen, where any nitrogen-bound $C_{1-6}$-alkyl is optionally substituted with hydroxy, $C_{1-6}$-alkoxy, $C_{2-6}$-alkenyloxy, amino, mono- and di($C_{1-6}$-alkyl)amino, carboxy, $C_{1-6}$-alkylcarbonylamino, halogen, $C_{1-6}$-alkylthio, $C_{1-6}$-alkyl-sulphonyl-amino or guanidine;

each $R_6$ and $R_7$ is independently selected from the group consisting of hydrogen, optionally substituted $C_{1}$-$_{6}$-alkyl, optionally substituted $C_{2}$-$_{6}$-alkenyl, optionally substituted $C_{4-6}$- alkadienyl, optionally substituted $C_{2}$-$_{6}$-alkynyl, optionally substituted $C_{1}$-$_{6}$-alkoxycarbonyl, optionally substituted $C_{1-6}$-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di($C_{1-6}$-alkyl)aminocarbonyl, amino- $C_{1-6}$-alkyl-aminocarbonyl and mono- and di($C_{1-6}$-alkyl)amino- $C_{1-6}$-alkyl-aminocarbonyl; or $R_6$ and $R_7$ may together form a five- or six-membered nitrogen-containing ring;

or a pharmaceutically acceptable derivative thereof, for use in the treatment of a kidney disease.

It is also an aspect of the present disclosure to provide a composition comprising a compound of formula (II):

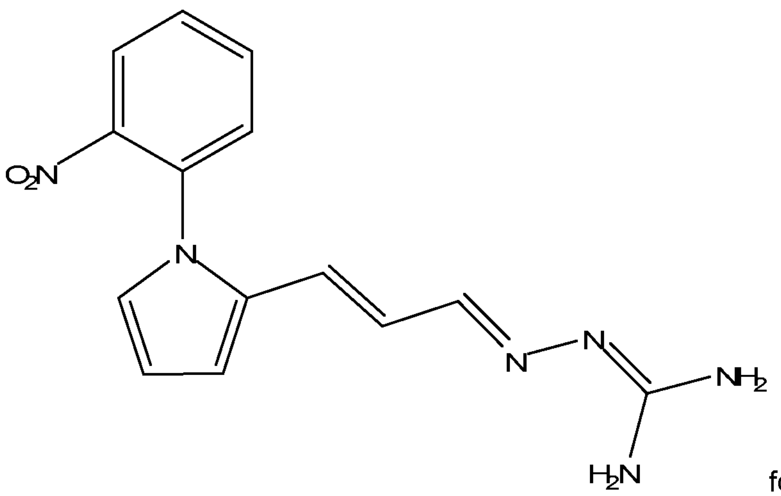

formula (II)

including tautomeric and isomeric forms, such as enantiomeric forms, diastereomeric forms and racemic forms, thereof; or a pharmaceutically acceptable derivative thereof, for use in the treatment of a kidney disease.

It is also an aspect of the present disclosure to provide a composition comprising a compound selected from the group consisting of {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; (*E*)-*N*-trans-{3-[1-(2-nitrophenyl)-1H-pyrrol-2-yl]-allylidene}-aminoguanidine; and (*E*)-*N*-trans-{3-[1-(2-nitrophenyl)-1H-pyrrol-2-yl]-allylidene}-aminoguanidinium acetate, or a pharmaceutically acceptable derivative thereof, for use in the treatment of a kidney disease.

In one embodiment of the present disclosure said kidney disease is a kidney disease that present with proteinuria.

In one embodiment of the present disclosure said kidney disease is a glomerular disease, such as a disease

affecting the podocytes of the glomeruli.

In one embodiment of the present disclosure said kidney disease is nephrotic syndrome (glomerulonephrosis), such as primary nephrotic syndrome or secondary nephrotic syndrome.

Definitions

The term "pharmaceutically acceptable derivative" in the present context includes pharmaceutically acceptable salts, which indicate a salt which is not harmful to the patient. Such salts include pharmaceutically acceptable basic or acid addition salts as well as pharmaceutically acceptable metal salts, ammonium salts and alkylated ammonium salts. A pharmaceutically acceptable derivative further includes esters and prodrugs, or other precursors of a compound which may be biologically metabolized into the active compound, or crystal forms of a compound.

The term "acid addition salt" is intended to include "pharmaceutically acceptable acid addition salt" which indicates salts which are not harmful to the patient. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 66, 2, (1977).

The term "therapeutically effective amount" of a compound as used herein refers to an amount sufficient to cure, alleviate, prevent, reduce the risk of, or partially arrest the clinical manifestations of a given disease or disorder and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The terms "treatment" and "treating" as used herein refer to the management and care of a patient for the purpose of combating a condition, disease or disorder. The term is intended to include the full spectrum of treatments for a given condition from which the patient is suffering. The patient to be treated is preferably a mammal, in particular a human being. Treatment of animals, such as mice, rats, dogs, cats, horses, cows, sheep and pigs, is, however, also within the scope of the present context. The patients to be treated can be of various ages.

**Description of Drawings**

Figure 1: Rats treated with test compound 1 (AP1189; (*(E)-N-trans*-{3-[1-(2-nitrophenyl)-1H-pyrrol-2-yl]-allylidene} -aminoguanidium acetate) have a significant reduction in proteinuria at day 28 and day 42 compared to control rats (vehicle). Control rats maintained an unchanged albumin excretion at all time points, whereas rats treated with test compound 1 show a 31±8% reduction in albumin excretion rate at day 28 and a 48±10% reduction in albumin excretion rate at day 482. See Examples for details.

Figure 2A) Rats treated with test compound 1 (AP1189; (*(E)-N-trans*-{3-[1-(2-nitrophenyl)-1H-pyrrol-2-yl]-allylidene} -aminoguanidium acetate) have a significantly higher GFR (creatinine clearance) following 4 weeks treatment than the rats treated with the positive control (ACTH 1-24):

i) positive control at 10 mg/day: 2.19 ±0.24 mL/min
ii) test compound 1 at 20 mg/kg: 3.12±0.14 mL/min (p=0.002)
iii) test compound 1 at 40 mg/kg: 2.94±0.14 mL/24 min (p=0.01).

Figure 2B) Rats treated with test compound 1 (AP1189; (*(E)-N-trans*-{3-[1-(2-nitrophenyl)-1H-pyrrol-2-yl]-allylidene} -aminoguanidium acetate) have a significantly reduced fraction albumin excretion (FEAlb) compared to positive control (ACTH 1-24):

i) positive control at 10 mg/day: 0.59±0.02%
ii) test compound 1 at 20 mg/kg: 0.045±0.04% (p=0.007)
iii) test compound 1 at 40 mg/kg: 0.35±0.05% (p=0.001).

## Detailed description

The invention is defined by the appended claims. Any embodiment not falling under the scope of the appended claims does not form part of the invention.

Melanocortin (MC) receptors (MC1 R-MC5R), a family of class A G protein-coupled receptors (GPCRs), are attractive therapeutic targets for a number of conditions due to their wide distribution and diversity of physiological processes they regulate. MC1R regulates UV light-induced skin tanning and other immune responses because of its expression on leukocytes. MC2R regulates cortisol production on the adrenal glands, whereas MC5R plays a role on exocrine glands secretions. MC3R and MC4R exert non-redundant functions on energy homeostasis in addition to specific anti-inflammatory roles; whereas MC3R activation is particularly protective for joint inflammation such as arthritis, MC4R provides neuroprotection in brain inflammation. Accordingly, an array of pathological situations could be targeted with MCR-drugs including skin conditions, cardiovascular pathologies, joint inflammation, obesity and cachexia.

Peripheral MC1R and MC3R can be pharmacologically activated to induce anti-inflammation. The endogenous agonist α-melanocyte-stimulating hormone (αMSH), like other protective mediators, is released by immune cells to counterbalance proinflammatory signals, thus preventing excessive tissue damage. In line with the resolution of inflammation concept, therapeutics targeting MC1R and MC3R act by mimicking the body's own protective resources and might be characterized by a lighter burden of side effects.

Shown to be effective in rheumatic diseases since the early 1950s, the use of corticotropin or adrenocorticotropin hormone (ACTH) declined when synthetic glucocorticoids became available. However, the discovery of an alternative anti-inflammatory mechanism for ACTH involving activation of peripheral MC receptors on immune cells has revived the interest in developing novel ACTH-like molecules with no steroidogenic effects for the treatment of joint diseases such as gout or RA (rheumatoid arthritis). However, the limitation in the translational delivery of novel MC drugs besides the marketed ACTH formulations is imposed by the lack of receptor selectivity achieved so far.

Innovative approaches in G protein-coupled receptor drug discovery might help to overcome this limitation. Allosteric modulation consists in the ability of a molecule to enhance (positive modulation) or reduce (negative modulation) the effect of the endogenous ligand by binding to a distinct site of the receptor protein, termed allosteric site. A higher degree of selectivity is expected as allosteric regions are less conserved among the five MCRs, and indeed, allosteric modulators at MC4R are currently under development for the treatment of obesity.

Another emerging concept of significant therapeutic interest is the one of biased agonism. The obsolete notion that receptors could exist in two unique conformations, the active one and the inactive one, has been replaced with the conception that multiple active conformations can exist, each one creating a distinct signal yielding multiple functional outcomes. Receptor activation, rather than linear and static, is emerging as a highly dynamic and multidimensional process in which a diversity of active conformations may be induced by different molecules leading to distinct effects.

The small molecule AP1189 (*(E)-N-trans*-{3-[1-(2-nitrophenyl)-1H-pyrrol-2-yl]-allylidene}-aminoguanidium acetate) has been characterized as a biased agonist at receptors MC1R and MC3R, which does not induce canonical cAMP generation, but cause ERK1/2 phosphorylation, a signaling responsible for the proefferocytic effect evoked in mouse primary macrophages. AP1189 was shown to reduce cytokine release in macrophages, whereas no melanogenesis was induced by AP1189 in melanocytes. In vivo, oral AP1189 elicits anti-inflammatory actions in peritonitis and accelerated the resolution phase, and afforded significant reduction of macroscopic and histological parameters of joint disruption in experimental inflammatory arthritis. AP1189 is thus a biased dual agonist at MC1 R and MC3R with anti-inflammatory properties together with a lack of effect on melanogenesis.

Melanocortin therapy by using adrenocorticotropic hormone (ACTH) or non-steroidogenic melanocortin peptides attenuates proteinuria and glomerular injury in experimental glomerular diseases and induces remission of nephrotic syndrome in patients with diverse glomerulopathies, even those resistant to steroids.

Proteinuria, the hallmark of glomerular injury, is a common finding on urinalysis, and is by itself a strong, independent and modifiable risk factor for end stage renal disease, premature death of cardiovascular origin, and ischemic stroke in patients with diabetes. Despite recent advances refractory proteinuria continues to be a challenge in clinical practice.

It is an aspect of the present disclosure to provide a composition comprising a compound of formula (I) or (Ia):

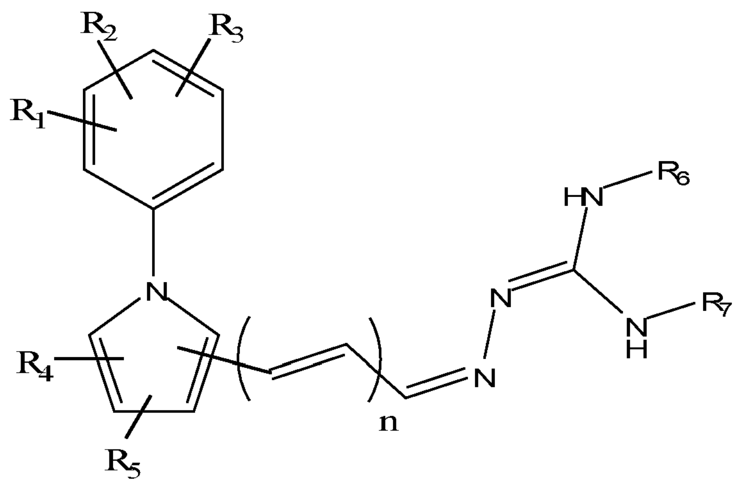

formula (I)

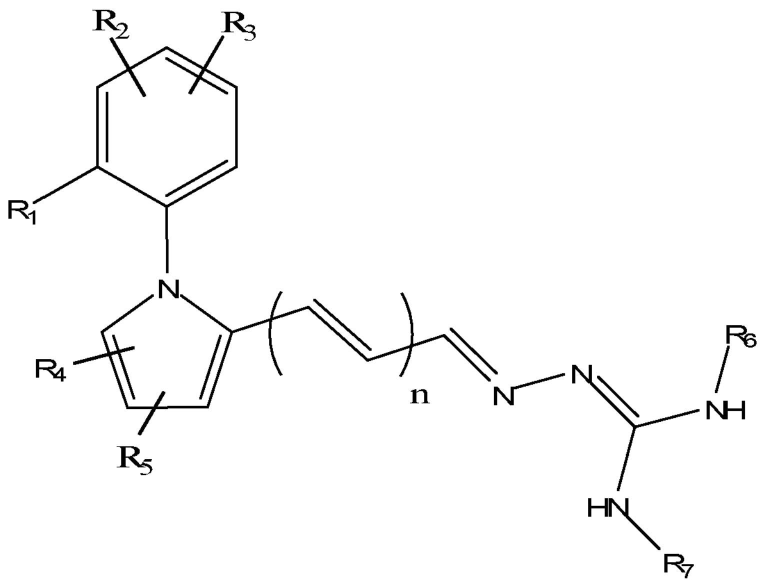

formula (Ia)

including tautomeric and isomeric forms, such as enantiomeric forms, diastereomeric forms and racemic forms, thereof; wherein

n is 1, 2 or 3;

each $R_1$, $R_2$, $R_3$, $R_4$ and $R_5$ is independently selected from the group consisting of hydrogen, optionally substituted $C_{1-6}$-alkyl, optionally substituted $C_{3-6}$-cycloalkyl, optionally substituted $C_{2-6}$-alkenyl, optionally substituted $C_{4-6}$-alkadienyl, optionally substituted $C_{2-6}$- alkynyl, hydroxy, optionally substituted $C_{1-6}$-alkoxy, optionally substituted $C_{2-6}$-alkenyloxy, carboxy, optionally substituted $C_{1-6}$-alkoxycarbonyl, optionally substituted $C_{1-6}$- alkylcarbonyl, formyl, $C_{1-6}$-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, mono-and di($C_{1-6}$-alkyl)amino, carbamoyl, mono- and di($C_{1-6}$-alkyl)aminocarbonyl, amino-$C_{1-6}$-alkyl-aminocarbonyl, mono- and di($C_{1-6}$- alkylamino-$C_{1-6}$-alkyl-aminocarbonyl, $C_{1-6}$-alkylcarbonylamino, amino-$C_{1-6}$-alkyl- carbonylamino, mono- and di($C_{1-6}$-alkyl)amino-$C_1$-$_6$-alkyl-carbonylamino, cyano, guanidino, carbamido, $C_{1-6}$-alkanoyloxy,

$C_{1-6}$-alkylsulphonyl, $C_{1-6}$-alkylsulphinyl, $C_{1-6}$-alkylsulphonyl- oxy, aminosulfonyl, mono- and di($C_{1-6}$-alkyl)aminosulfonyl, nitro, optionally substituted $C_{1-6}$-alkylthio and halogen, where any nitrogen-bound $C_{1-6}$-alkyl is optionally substituted with hydroxy, $C_{1-6}$-alkoxy, $C_{2-6}$-alkenyloxy, amino, mono- and di($C_{1-6}$-alkyl)amino, carboxy, $C_{1-6}$-alkylcarbonylamino, halogen, $C_{1-6}$-alkylthio, $C_{1-6}$-alkyl-sulphonyl-amino or guanidine;

each $R_6$ and $R_7$ is independently selected from the group consisting of hydrogen, optionally substituted $C_{1-6}$-alkyl, optionally substituted $C_{2-6}$-alkenyl, optionally substituted $C_{4-6}$- alkadienyl, optionally substituted $C_{2-6}$-alkynyl, optionally substituted $C_{1-6}$-alkoxycarbonyl, optionally substituted $C_{1-6}$-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di($C_{1-6}$-alkyl)aminocarbonyl, amino- $C_{1-6}$-alkyl-aminocarbonyl and mono- and di($C_{1-6}$-alkyl)amino- $C_{1-6}$-alkyl-aminocarbonyl; or $R_6$ and $R_7$ may together form a five- or six-membered nitrogen-containing ring;
or a pharmaceutically acceptable derivative thereof, for use in the treatment of a kidney disease.

It is also an aspect of the present disclosure to provide use of a composition comprising a compound of formula (I) or (Ia):

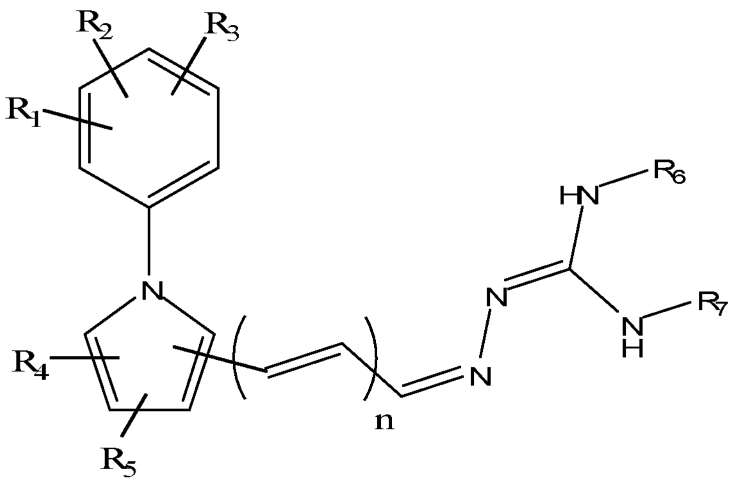

formula (I)

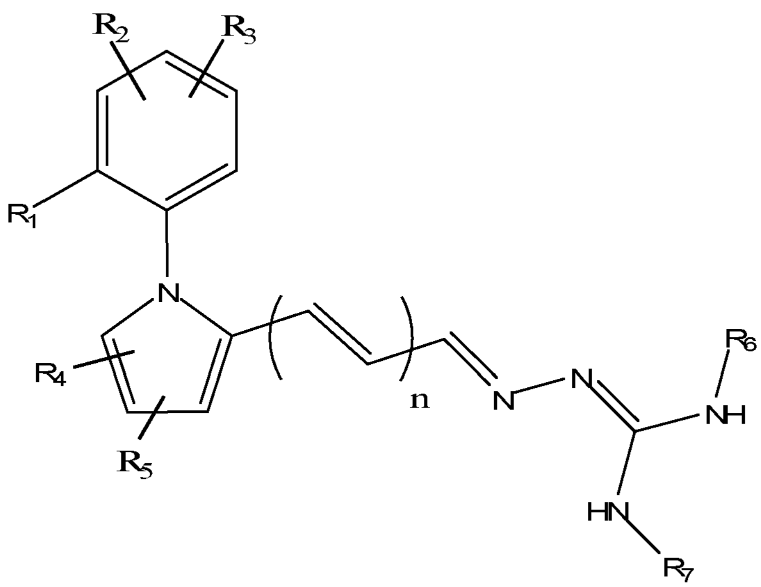

formula (Ia)

including tautomeric and isomeric forms, such as enantiomeric forms, diastereomeric forms and racemic forms, thereof;

wherein each of n, $R_1$, $R_2$, $R_3$, $R_4$ and $R_5$, $R_6$ and $R_7$ are as defined herein above, or a pharmaceutically acceptable derivative thereof, for the manufacture of a medicament for the treatment of a kidney disease.

It is also an aspect of the present disclosure to provide a method for treating a kidney disease, said method comprising one or more steps of administration of a composition comprising a compound of formula (I) or (Ia):

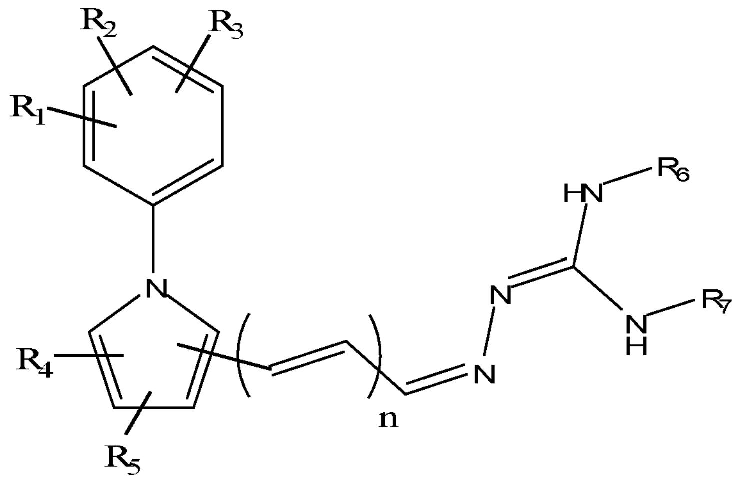

formula (I)

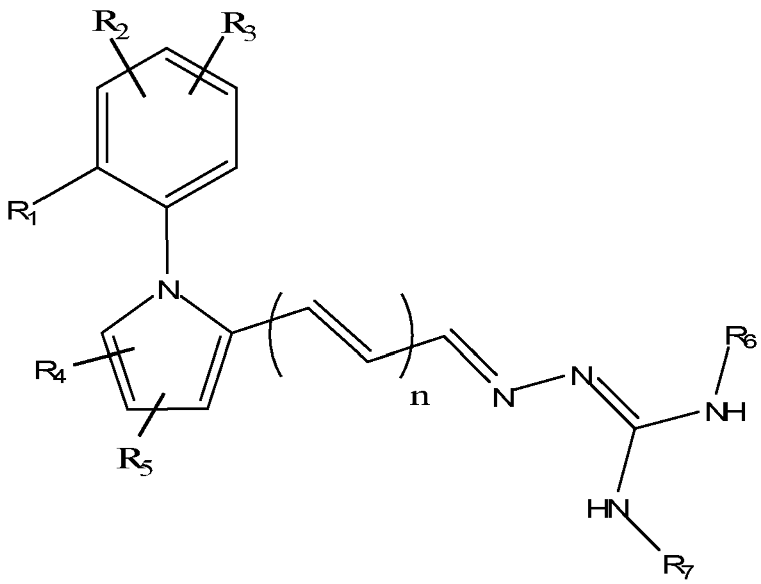

formula (Ia)

including tautomeric and isomeric forms, such as enantiomeric forms, diastereomeric forms and racemic forms, thereof; wherein each of n, $R_1$, $R_2$, $R_3$, $R_4$ and $R_5$, $R_6$ and $R_7$ are as defined herein above, or a pharmaceutically acceptable derivative thereof, to an individual in need thereof.

The present disclosure also provides a composition comprising a compound of formula (II):

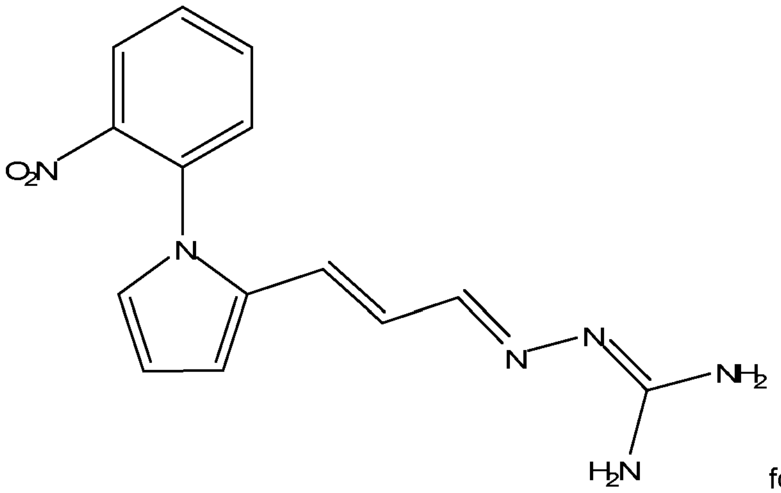

formula (II)

including tautomeric and isomeric forms, such as enantiomeric forms, diastereomeric forms and racemic forms, thereof; or a pharmaceutically acceptable derivative thereof, for use in the treatment of a kidney disease.

In one embodiment of the present disclosure there is provided a composition comprising a compound of formula (I), (Ia) or (II) as defined herein for use in the treatment of a kidney disease, wherein said kidney disease present with proteinuria.

Proteinuria is the presence of excess proteins in the urine. In healthy persons, urine contains very little protein; an excess is suggestive of illness. The main mechanisms to cause proteinuria is disease in the kidney (esp. the glomerulus) or kidney damage, increased quantity of proteins in serum (overflow proteinuria), low reabsorption at proximal tubule, certain biological agents and excessive fluid intake.

Proteinuria may be defined as defined as >3.5 g per 1.73 $m^2$ body surface area per day, or > 40 mg per square meter body surface area per hour in children.

In one embodiment of the present disclosure there is provided a composition comprising a compound of formula (I), (Ia) or (II) as defined herein for use in the treatment of a kidney disease, wherein said kidney disease present with proteinuria defined as >3.5 g per 1.73 $m^2$ body surface area per day, or > 40 mg per square meter body surface area per hour in children.

A 'kidney disease presenting with proteinuria' means that proteinuria is an associated symptom or sign of said kidney disease. A 'kidney disease presenting with proteinuria' may also be described as a kidney disease associated with proteinuria or a kidney disease with proteinuria or a proteinuric kidney disease.

In one embodiment of the present disclosure there is provided a composition comprising a compound of formula (I), (Ia) or (II) as defined herein for use in the treatment of a kidney disease, wherein said kidney disease is a glomerular disease, such as a disease affecting the podocytes of the glomeruli.

A glomerular disease may also reflect any type of glomerular injury caused by glomerular disease or any other stimuli, such as other underlying diseases or influences.

The term 'caused by' a disease in the present context means that the injury arises or emerges as a cause or result of a disease, such as an underlying disease, i.e. as a result of the pathologic condition.

In one embodiment of the present disclosure there is provided a composition comprising a compound of formula (I), (Ia) or (II) as defined herein for use in the treatment of a kidney disease, wherein said kidney disease is nephrotic syndrome (glomerulonephrosis).

Nephrotic syndrome is a collection of symptoms due to kidney damage, including proteinuria, low blood albumin levels, high blood lipids, and significant swelling. Other symptoms may include weight gain, feeling tired, and foamy urine. Complications may include blood clots, infections, and high blood pressure. Causes include a number of kidney diseases and may also occur as a complication of for example diabetes or lupus. The underlying mechanism typically involves damage to the glomeruli of the kidney.

In one embodiment of the present disclosure there is provided a composition comprising a compound of formula (I), (Ia) or (II) as defined herein for use in the treatment of a kidney disease, wherein said kidney disease is primary nephrotic syndrome (primary glomerulonephrosis).

In one embodiment of the present disclosure said primary nephrotic syndrome is membranous glomerulonephritis (MGN) (or membranous nephropathy (MN)).

In one embodiment of the present disclosure said primary nephrotic syndrome is focal segmental glomerulosclerosis (FSGS)

In one embodiment of the present disclosure said primary nephrotic syndrome is membranoproliferative glomerulonephritis (MPGN) (mesangiocapillary glomerulonephritis).

In one embodiment of the present disclosure said membranoproliferative glomerulonephritis (MPGN) is selected from Type 1 MPGN and Type 2 MPGN.

In one embodiment of the present disclosure said primary nephrotic syndrome is rapidly progressive glomerulonephritis (RPGN) (crescentic GN).

In one embodiment of the present disclosure said primary nephrotic syndrome is minimal change disease (MCD).

In one embodiment of the present disclosure there is provided a composition comprising a compound of formula (I), (Ia) or (II) as defined herein for use in the treatment of a kidney disease, wherein said kidney disease is secondary nephrotic syndrome (secondary glomerulonephrosis).

In one embodiment of the present disclosure said secondary nephrotic syndrome is caused by an underlying disease (or simply caused by a disease). The term 'caused by' a disease in the present context means that the nephrotic syndrome arises or emerges as a cause or result of a disease, such as an underlying disease, i.e. as a result of the pathologic condition.

In one embodiment of the present disclosure said secondary nephrotic syndrome is caused by an underlying autoimmune disease.

In one embodiment of the present disclosure said secondary nephrotic syndrome is caused by an underlying cancer disease.

In one embodiment of the present disclosure said secondary nephrotic syndrome is caused by an underlying genetic disorder.

In one embodiment of the present disclosure said secondary nephrotic syndrome is caused by an underlying disease selected from the group consisting of: Systemic lupus erythematosus (SLE), Diabetic nephropathy, Sarcoidosis, Sjögren's syndrome, Amyloidosis, Multiple myeloma, Vasculitis, Cancer and Genetic disorders (such as congenital nephrotic syndrome).

Lupus nephritis is an inflammation of the kidneys caused by SLE. Hence, in one embodiment of the present disclosure the secondary nephrotic syndrome is caused by lupus nephritis.

In one embodiment of the present disclosure said secondary nephrotic syndrome is caused by Diabetic nephropathy.

In one embodiment of the present disclosure said secondary nephrotic syndrome is caused by an infection.

In one embodiment of the present disclosure said secondary nephrotic syndrome is caused by a urinary tract infection.

In one embodiment of the present disclosure said secondary nephrotic syndrome is caused by an infection selected from the group consisting of HIV, syphilis, hepatitis such as hepatitis A, B and C, post-streptococcal infection, urinary schistosomiasis and Ebola.

In one embodiment of the present disclosure said secondary nephrotic syndrome is drug-induced; i.e. a drug-induced nephrotic syndrome.

In one embodiment of the present disclosure said drug-induced nephrotic syndrome is caused by a drug selected from the group consisting of antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (such as heroin).

The term 'caused by' a drug in the present context means that the nephrotic syndrome arises or emerges as a cause or result of the use or administration of a drug.

In one embodiment of the present disclosure there is provided a composition comprising a compound of formula (I), (Ia) or (II) as defined herein for use in the treatment of a kidney disease, wherein said kidney disease is an inflammatory kidney disease.

In one embodiment of the present disclosure there is provided a composition comprising a compound of formula (I), (Ia) or (II) as defined herein for use in the treatment of a kidney disease, wherein said kidney disease is glomerulonephritis (GN).

In one embodiment of the present disclosure said glomerulonephritis is selected from the group consisting of IgA nephropathy (Berger's disease), IgM nephropathy, Post-infectious glomerulonephritis and Thin basement membrane disease.

In one embodiment of the present disclosure there is provided a composition comprising a compound of formula (I) or (Ia) as defined herein for use in the treatment of a kidney disease, wherein n = 1.

In one embodiment of the present disclosure there is provided a composition comprising a compound of formula (I) or (Ia) as defined herein for use in the treatment of a kidney disease, wherein each $R_1$, $R_2$ and $R_3$ is independently selected from the group consisting of -H, $-CH_3$, $-CF_3$, $-CCl_3$, -O-$C_{1-6}$ alkyl, -OH, $-OCH_3$, -NH-$C_{1-6}$ alkyl, - N($C_{1-6}$ alkyl)$_2$, -S-$C_{1-6}$ alkyl, -$^t$Bu, -CN, $-SO_3$-$C_{1-6}$ alkyl, $-NH_2$, $-NO_2$, $-CO_2H$, $-CO_2$-$C_{1-6}$ alkyl, -C(=O)-$C_{1-6}$ alkyl, -CHO, morpholine, pyrrolidine, pyrrole, or halogen.

In one embodiment of the present disclosure there is provided a composition comprising a compound of formula

(I) or (Ia) as defined herein for use in the treatment of a kidney disease, wherein $R_1$ and/or $R_2$ each is an electron-withdrawing group.

An electron-withdrawing group is understood as an electron-withdrawing element or functional group that draws electron density from neighbouring atoms towards itself, usually by resonance or inductive effects. Whether a functional group is electron-withdrawing or electron-donating can be determined using the Hammett equation, an equation known to the person skilled in the art. Hammett substituent constants, also known as substituent constants $\sigma$ can be used as a measure of a functional group's ability to draw electron density from neighboring atoms towards itself. An electron-withdrawing group is an electron-withdrawing element or functional group with a substituent constant $\sigma > 0$, such as between 0.01 and 0.9 for example 0.78. These values are known in the art and can be found in tables in the scientific literature such as J. Org. Chem., 23, 420 (1958). $CF_3$, $CCl_3$, F, Cl, CN and $NO_2$ are examples of electron-withdrawing groups.

In one embodiment of the present disclosure said $R_1$ is selected from the group consisting of $CF_3$, $CCl_3$, F, Cl, CN and $NO_2$.

In one embodiment of the present disclosure said $R_2$ and/or $R_3$ each is hydrogen.

In one embodiment of the present disclosure each $R_4$ and $R_5$ is independently selected from the group consisting of -H, $C_{1-6}$ alkyl, halogen, -O($C_{1-6}$ alkyl), -NH($C_{1-6}$ alkyl) and - C(=O)$C_{1-6}$ alkyl.

In one embodiment of the present disclosure each $R_6$ and $R_7$ is independently selected from the group consisting of hydrogen, methyl, ethyl and propyl.

In one embodiment of the present disclosure said $R_6$ and $R_7$ are each hydrogen.

In one embodiment of the present disclosure, said $R_6$ is hydrogen and $R_7$ is methyl or ethyl. In one embodiment of the present disclosure, said $R_6$ is hydrogen and $R_7$ is methyl. In one embodiment of the present disclosure, said $R_6$ is hydrogen and $R_7$ is ethyl. In one embodiment of the present disclosure, said $R_6$ and $R_7$ are both methyl. In one embodiment of the present disclosure, said $R_6$ and $R_7$ are both ethyl.

In one embodiment of the present disclosure, said $R_2$, $R_3$, $R_4$, $R_5$, $R_6$ and $R_7$ are each hydrogen, and said $R_1$ is selected from the group consisting of $CF_3$, $CCl_3$, F, Cl, $NO_2$ and CN.

In one embodiment of the present disclosure, said $R_2$, $R_3$, $R_4$, $R_5$, $R_6$ and $R_7$ are each hydrogen, said $R_1$ is selected from the group consisting of $CF_3$, $CCl_3$, F, Cl, $NO_2$ or CN, and n = 1.

In one embodiment of the present disclosure said $R_3$ is located on the 4-position.

In one embodiment of the present disclosure, said $R_3$ is selected from the group consisting of F, Cl and Br.

In one embodiment of the present disclosure, said compound is {3-[1-(2-nitrophenyl)-1 H-pyrrol-2-yl]-allylidene} -aminoguanidine, preferably (*E*)-*N*-*trans*-{3-[1-(2-nitrophenyl)-1H-pyrrol-2-yl]-allylidene}-aminoguanidine or a pharmaceutically acceptable salt thereof.

In one embodiment of the present disclosure there is provided a composition comprising a compound of formula (I), (Ia) or (II) as defined herein for use in the treatment of a kidney disease, wherein the pharmaceutically acceptable derivative thereof is a pharmaceutically acceptable salt of an inorganic acid or an organic acid.

In one embodiment of the present disclosure the organic acid is selected from the group consisting of: formic acid, acetic acid, trichloroacetic acid, trifluoroacetic acid, propionic acid, benzoic acid, cinnamic acid, citric acid, fumaric acid, glycolic acid, lactic acid, maleic acid, malic acid, malonic acid, mandelic acid, oxalic acid, picric acid, pyruvic acid, salicylic acid, succinic acid, methanesulfonic acid, ethanesulfonic acid, tartaric acid, ascorbic acid, pamoic acid, bismethylene salicylic acid, ethanedisulfonic acid, gluconic acid, citraconic acid, aspartic acid, stearic acid, palmitic acid, EDTA, glycolic acid, p-aminobenzoic acid, glutamic acid, benzenesulfonic acid and p-toluenesulfonic acid.

In one embodiment of the present disclosure said organic acid is acetic acid, succinic acid, tartaric acid or propionic acid.

In one embodiment of the present disclosure said inorganic acid is selected from the group consisting of: hydrochloric acid, hydrobromic acid, hydroiodic acid, phosphoric acid, sulphuric acid and nitric acid.

In one embodiment of the present disclosure said pharmaceutically acceptable acid is acetic acid.

In one embodiment of the present disclosure said compound is {3-[1-(2-nitrophenyl)-1H-pyrrol-2-yl]-allylidene} - aminoguanidinium acetate, preferably (*E*)-*N*-trans-{3-[1-(2-nitrophenyl)-1H-pyrrol-2-yl]-allylidene}-aminoguanidinium acetate.

In one embodiment of the present disclosure there is provided a composition comprising a compound of formula (Ia), wherein said $R_2$, $R_3$, $R_4$ and $R_5$ are each hydrogen, $R_6$ and $R_7$ are each independently selected from the group consisting of hydrogen, methyl, ethyl and propyl, said $R_1$ is selected from the group consisting of $CF_3$, $CCl_3$, F, Cl, $NO_2$ or CN, and n = 1 for use in the treatment of a kidney disease.

In one embodiment of the present disclosure there is provided a composition comprising a compound of formula (Ia), wherein said $R_2$, $R_3$, $R_4$, $R_5$, $R_6$ and $R_7$ are each hydrogen, said $R_1$ is selected from the group consisting of $CF_3$, $CCl_3$, F, Cl, $NO_2$ or CN, and n = 1 for use in the treatment of a kidney disease.

In one embodiment of the present disclosure there is provided a composition comprising a compound of formula (Ia), wherein said $R_2$, $R_3$, $R_4$, $R_5$, $R_6$ and $R_7$ are each hydrogen, said $R_1$ is selected from the group consisting of $CF_3$,

$CCl_3$, F, Cl, $NO_2$ or CN, and n = 1 for use in the treatment of a kidney disease, wherein said kidney disease present with proteinuria.

In one embodiment of the present disclosure there is provided a composition comprising a compound of formula (Ia), wherein said $R_2$, $R_3$, $R_4$, $R_5$, $R_6$ and $R_7$ are each hydrogen, said $R_1$ is selected from the group consisting of $CF_3$, $CCl_3$, F, Cl, $NO_2$ or CN, and n = 1 for use in the treatment of nephrotic syndrome. In one embodiment of the present disclosure said nephrotic syndrome is selected from the group consisting of primary nephrotic syndrome and secondary nephrotic syndrome.

In one embodiment of the present disclosure there is provided a composition comprising a compound of formula (Ia), wherein said $R_2$, $R_3$, $R_4$, $R_5$, $R_6$ and $R_7$ are each hydrogen, said $R_1$ is selected from the group consisting of $CF_3$, $CCl_3$, F, Cl, $NO_2$ or CN, and n = 1 for use in the treatment of primary nephrotic syndrome (primary glomerulonephrosis), wherein said primary nephrotic syndrome is selected from the group consisting of: membranous glomerulonephritis (MGN) (or membranous nephropathy (MN)), focal segmental glomerulosclerosis (FSGS), membranoproliferative glomerulonephritis (MPGN) (mesangiocapillary glomerulonephritis) selected from the group consisting of Type 1 MPGN and Type 2 MPGN, rapidly progressive glomerulonephritis (RPGN) (crescentic GN) and minimal change disease (MCD) .

In one embodiment of the present disclosure there is provided a composition comprising a compound of formula (Ia), wherein said $R_2$, $R_3$, $R_4$, $R_5$, $R_6$ and $R_7$ are each hydrogen, said $R_1$ is selected from the group consisting of $CF_3$, $CCl_3$, F, Cl, $NO_2$ or CN, and n = 1 for use in the treatment of secondary nephrotic syndrome (secondary glomerulonephrosis), wherein said secondary nephrotic syndrome is caused by a disease selected from the group consisting of: an underlying autoimmune disease, Systemic lupus erythematosus (lupus nephritis), Diabetic nephropathy, Sarcoidosis, Sjögren's syndrome, Amyloidosis, Multiple myeloma, Vasculitis, Cancer, Genetic disorders (such as congenital nephrotic syndrome), an infection and Diabetic nephropathy.

In one embodiment of the present disclosure there is provided a composition comprising a compound of formula (Ia), wherein said $R_2$, $R_3$, $R_4$, $R_5$, $R_6$ and $R_7$ are each hydrogen, said $R_1$ is selected from the group consisting of $CF_3$, $CCl_3$, F, Cl, $NO_2$ or CN, and n = 1 for use in the treatment of drug-induced nephrotic syndrome. In one embodiment of the present disclosure said drug-induced nephrotic syndrome is caused by a drug selected from the group consisting of: antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (especially heroin).

In one embodiment of the present disclosure there is provided a composition comprising a compound of formula (Ia), wherein said $R_2$, $R_3$, $R_4$, $R_5$, $R_6$ and $R_7$ are each hydrogen, said $R_1$ is selected from the group consisting of $CF_3$, $CCl_3$, F, Cl, $NO_2$ or CN, and n = 1 for use in the treatment of a kidney disease, wherein said kidney disease is selected from the group consisting of: a glomerular disease, a disease affecting the podocytes of the glomeruli, an inflammatory kidney disease and glomerulonephritis (GN); such as a glomerulonephritis selected from the group consisting of: IgA nephropathy (Berger's disease), IgM nephropathy, Post-infectious glomerulonephritis and Thin basement membrane disease.

In one embodiment of the present disclosure there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; (*E*)-*N*-*trans*-{3-[1-(2-nitrophenyl)-1H-pyrrol-2-yl]-allylidene} -aminoguanidine; and (*E*)-*N*-trans-{3-[1-(2-nitrophenyl)-1H-pyrrol-2-yl]-allylidene}-aminoguanidinium acetate, or a pharmaceutically acceptable derivative thereof, for use in the treatment of a kidney disease. In one embodiment of the present disclosure said kidney disease present with proteinuria.

In one embodiment of the present disclosure there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)-N-trans*-{3-[1-(2-nitrophenyl)-1H-pyrrol-2-yl]-allylidene} -aminoguanidine; and (*E*)-*N*-*trans*-{3-[1-(2-nitrophenyl)-1H-pyrrol-2-yl]-allylidene}-aminoguanidinium acetate, or a pharmaceutically acceptable derivative thereof,for use in the treatment of nephrotic syndrome. In one embodiment of the present disclosure said nephrotic syndrome is selected from the group consisting of primary nephrotic syndrome and secondary nephrotic syndrome.

In one embodiment of the present disclosure there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; (*E*)-*N*-*trans*-{3-[1-(2-nitrophenyl)-1H-pyrrol-2-yl]-allylidene} -aminoguanidine; and (*E*)-*N*-*trans*-{3-[1-(2-nitrophenyl)-1H-pyrrol-2-yl]-allylidene}-aminoguanidinium acetate, or a pharmaceutically acceptable derivative thereof, for use in the treatment of primary nephrotic syndrome (primary glomerulonephrosis), wherein said primary nephrotic syndrome is selected from the group consisting of: membranous glomerulonephritis (MGN) (or membranous nephropathy (MN)), focal segmental glomerulosclerosis (FSGS), membranoproliferative glomerulonephritis (MPGN) (mesangiocapillary glomerulonephritis) selected from the group consisting of Type 1 MPGN and Type 2 MPGN, rapidly progressive glomerulonephritis (RPGN) (crescentic GN) and minimal change disease (MCD) .

In one embodiment of the present disclosure there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; (*E*)-*N-trans*-{3-[1-(2-nitrophenyl)-1H-pyrrol-2-yl]-allylidene}-aminoguanidine; and *(E)-N-trans*-{3-[1-(2-nitrophenyl)-1H-pyrrol-2-yl]-allylidene}-aminoguanidinium acetate, or a pharmaceutically acceptable derivative thereof, for use in the treatment of secondary nephrotic syndrome (secondary glomerulonephrosis), wherein said secondary nephrotic syndrome is caused by a disease selected from the group consisting of: an underlying autoimmune disease, Systemic lupus erythematosus (lupus nephritis), Diabetic nephropathy, Sarcoidosis, Sjögren's syndrome, Amyloidosis, Multiple myeloma, Vasculitis, Cancer, Genetic disorders (such as congenital nephrotic syndrome), an infection and Diabetic nephropathy.

In one embodiment of the present disclosure there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)-N-trans*-{3-[1-(2-nitrophenyl)-1H-pyrrol-2-yl]-allylidene}-aminoguanidine; and (*E*)-*N-trans*-{3-[1-(2-nitrophenyl)-1H-pyrrol-2-yl]-allylidene}-aminoguanidinium acetate, or a pharmaceutically acceptable derivative thereof, for use in the treatment of secondary nephrotic syndrome caused by Diabetic nephropathy.

In one embodiment of the present disclosure there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)-N-trans*-{3-[1-(2-nitrophenyl)-1H-pyrrol-2-yl]-allylidene}-aminoguanidine; and (*E*)-*N-trans*-{3-[1-(2-nitrophenyl)-1H-pyrrol-2-yl]-allylidene}-aminoguanidinium acetate, or a pharmaceutically acceptable derivative thereof, for use in the treatment of drug-induced nephrotic syndrome. In one embodiment of the present disclosure said drug-induced nephrotic syndrome is caused by a drug selected from the group consisting of: antibiotics, nonsteroidal anti-inflammatory drugs (NSAID), radiocontrast media, anticancer drugs, antirheumatic drugs, antibody-based therapies, anti-TNF-a therapies, penicillamine, nicotine, lithium carbonate, gold and other heavy metals, ACE inhibitors and opiates (especially heroin).

In one embodiment of the present disclosure there is provided a composition comprising a compound selected from a compound of formula (II), {3-[1-(2-nitrophenyl)-1H-pyrrol-2-yl]-allylidene}-aminoguanidine; {3-[1-(2-nitrophenyl)-1H-pyrrol-2-yl]-allylidene}-aminoguanidinium acetate; *(E)-N-trans*-{3-[1-(2-nitrophenyl)-1H-pyrrol-2-yl]-allylidene}-aminoguanidine; and *(E)-N*-trans-{3-[1-(2-nitrophenyl)-1H-pyrrol-2-yl]-allylidene}-aminoguanidinium acetate, or a pharmaceutically acceptable derivative thereof, for use in the treatment of a kidney disease selected from the group consisting of: a glomerular disease, a disease affecting the podocytes of the glomeruli, an inflammatory kidney disease and glomerulonephritis (GN); such as a glomerulonephritis selected from the group consisting of: IgA nephropathy (Berger's disease), IgM nephropathy, Post-infectious glomerulonephritis and Thin basement membrane disease.

Without wishing to be bound to any theory, the compounds of the present disclosure in one embodiment of the present disclosure exert their beneficial effects via an effect as an agonist of one or more MC receptors, such as via the MC1R and MC3R, such as via MC1R. The compounds of the present disclosure in one embodiment of the present disclosure exert their beneficial effects via an effect as a biased agonist of the MC1R and MC3R.

In one embodiment of the present disclosure there is provided a composition comprising a compound of formula (I), (Ia) or (II) as defined herein, including tautomeric and isomeric forms, such as enantiomeric forms, diastereomeric forms and racemic forms, thereof, for use in the treatment of a kidney disease, wherein said compound is:

i) an agonist of one or more MC receptors,
ii) an agonist of the MC1R and MC3R,
iii) an agonist of MC1R,
iv) an agonist of the MC1R and/or MC3R, but not an agonist of MC2R and MC4R,
v) a biased agonist of the MC1R and MC3R,
vi) a biased agonist of the MC1R and MC3R, that mainly induces ERK1/2 phosphorylation, and insignificantly induces cAMP generation,
vii) a biased agonist of the MC1R and MC3R, that does not stimulate melanogenesis via the MC1R, and/or
viii) an agonist of the MC1R and/or MC3R, with no effect on energy homeostasis such as food intake

In one embodiment of the present disclosure the compound of the present disclosure does not reduce food intake, such as does not reduce food intake via the MC3R and/or the MC4R.

In one embodiment of the present disclosure the compound of the present disclosure does not induce melanogenesis.

## Combination therapies

The compounds or compositions of the present disclosure may be combined with or comprise one or more

additional active ingredients which are understood as other therapeutically effective compounds or pharmaceutically acceptable derivatives thereof.

In one embodiment of the present disclosure the composition comprising a compound of formula (I), (Ia) or (II), or a pharmaceutically acceptable derivative thereof, comprises, separately or together, one or more additional active pharmaceutical ingredients.

In one embodiment of the present disclosure the composition comprising a compound of formula (I), (Ia) or (II), or a pharmaceutically acceptable derivative thereof, comprises, separately or together, one or more additional active pharmaceutical ingredients used for the treatment of kidney disease.

In one embodiment of the present disclosure said one or more additional active pharmaceutical ingredients comprise ingredients used for the treatment of kidney disease.

In one embodiment of the present disclosure said one or more additional active pharmaceutical ingredients comprise ingredients used for the treatment of the underlying cause of said kidney disease.

In one embodiment of the present disclosure said one or more additional active pharmaceutical ingredients comprise ingredients used for the treatment of the underlying cause of said kidney disease, such as ingredients for treatment of high blood pressure, of high blood cholesterol, and of infection; a low salt diet and limiting fluids.

In one embodiment of the present disclosure said one or more additional active pharmaceutical ingredients used for the treatment of kidney disease is selected from the group consisting of angiotensin converting enzyme (ACE) inhibitors, aldosterone antagonist and angiotensin receptor blocker (ARB).

In one embodiment of the present disclosure said one or more additional active pharmaceutical ingredients used for the treatment of kidney disease and proteinuria secondary to autoimmune disease is treated with steroids or a steroid-sparing agent plus the use of ACE inhibitors.

In another embodiment of the present disclosure said one or more additional active pharmaceutical ingredients comprise ingredients used for the treatment of the underlying cause of the kidney disease.

In one embodiment of the present disclosure the combination of additional medicaments has a dose-sparing effect of lowering the required dosage of the medication used in combination with the compound of the present disclosure.

In one embodiment of the present disclosure the composition comprising a compound of formula (I), (Ia) or (II) as defined herein, and the additional active ingredient, are administered simultaneously, sequentially or separately.

In one embodiment of the present disclosure the composition comprising a compound of formula (I), (Ia) or (II) as defined herein is administered before the additional active ingredient. In another embodiment of the present disclosure, the composition comprising a compound of formula (I) as defined herein is administered simultaneously with the additional active ingredient. In yet another embodiment of the present disclosure, the composition comprising a compound of formula (I) as defined herein is administered after the additional active ingredient.

## Routes of administration

It will be appreciated that the preferred route of administration will depend on the general condition and age of the subject to be treated, the nature of the condition to be treated, the location of the tissue to be treated in the body and the active ingredient chosen.

In one embodiment of the present disclosure the composition comprising a compound of formula (I), (Ia) or (II) as defined herein is administered by systemic administration, local administration, enteral administration or parenteral administration. Appropriate dosage forms for such administration may be prepared by conventional techniques.

### *Systemic administration*

Systemic administration is capable of introducing the compound into the blood stream to ultimately target the sites of desired action.

Such routes of administration are any suitable routes, such as an enteral route, the oral, rectal, nasal, pulmonary, buccal, sublingual, transdermal, intracisternal, intraperitoneal, and parenteral (including subcutaneous, intramuscular, intrathecal, intravenous and intradermal) route.

In one embodiment of the present disclosure, the composition comprising a compound of formula (I), (Ia) or (II) as defined herein is administered by systemic administration.

### *Oral administration*

Oral administration is normally for enteral drug delivery, wherein the compound is delivered through the enteral mucosa. Syrups and solid oral dosage forms, such as tablets, capsules and the like, are commonly used.

In one embodiment of the present disclosure, the composition comprising a compound of formula (I), (Ia) or (II) as defined herein is administered by oral administration.

*Parenteral administration*

Parenteral administration is any administration route not being the oral/enteral route whereby the medicament avoids first-pass degradation in the liver. Accordingly, parenteral administration includes any injections and infusions, for example bolus injection or continuous infusion, such as intravenous administration, intramuscular administration, subcutaneous administration. Furthermore, parenteral administration includes inhalations and topical administration.
Accordingly, the compound may be administered topically to cross any mucosal membrane of an animal to which the biologically active substance is to be given, e.g. in the nose, vagina, eye, mouth, genital tract, lungs, gastrointestinal tract, or rectum, preferably the mucosa of the nose, or mouth, and accordingly, parenteral administration may also include buccal, sublingual, nasal, rectal, vaginal and intraperitoneal administration as well as pulmonal and bronchial administration by inhalation or installation. Also, the compound may be administered topically to cross the skin.
The subcutaneous and intramuscular forms of parenteral administration are generally preferred.

*Local treatment*

The compound as disclosed herein is in one embodiment of the present disclosure used as a local treatment, i.e. is introduced directly to the site(s) of action. Accordingly, the compound may be applied to the skin or mucosa directly, or the compound may be injected into the site of action, for example into the diseased tissue or to an end artery leading directly to the diseased tissue.

Dosage

According to the present disclosure, the composition comprising a compound of formula (I), (Ia) or (II) is administered to individuals in need of treatment in pharmaceutically effective doses. A therapeutically effective amount of a compound is an amount sufficient to cure, prevent, reduce the risk of, alleviate or partially arrest the clinical manifestations of a given disease and its complications. The amount that is effective for a particular therapeutic purpose will depend on the severity and the sort of the disorder as well as on the weight and general state of the subject. The compound may be administered one or several times per day, such as from 1 to 8 times per day, such as from 1 to 6 times per day, such as from 1 to 5 times per day, such as from 1 to 4 times per day, such as from 1 to 3 times per day, such as from 1 to 2 times per day, such as from 2 to 4 times per day, such as from 2 to 3 times per day. Alternatively, the compound may be administered less than once a day, for example once a day, such as once every second day, for example once every third day, such as once every fourth day, for example once every fifth day, such as once every sixth day, for example once every week.
In one embodiment of the present disclosure the composition comprising a compound of formula (I) as defined herein is administered in a therapeutically effective amount, such as in an amount of 1 mg to 1000 mg compound of formula (I), (Ia) or (II) per day.
It follows that in one embodiment of the present disclosure the compound is administered in an amount of 1 mg to 1000 mg, such as 1 to 5 mg, 5 to 10 mg, 10 to 15 mg, 15 to 20 mg, 20 mg, 20 to 30 mg, 30 to 60 mg, 60 to 80 mg, 80 to 100 mg, 100 to 130 mg, 130 to 160 mg, 160 to 200 mg, 200 to 240 mg, 240 to 280 mg, 280 to 320 mg, 320 to 360 mg, 360 to 400 mg, 400 to 440 mg, 440 to 500 mg, 500 to 560 mg, 560 to 620 mg, 620 to 680 mg, 680 to 740 mg, 740 to 800 mg, 800 to 860 mg, 860 to 920 mg, 920 to 980 mg, 980 to 1000 mg, for example 500 to 1000 mg per day.
Per day means the dosage may be given in one dosage or divided in multiple dosages per day, including once a day (QD), twice a day (BID) and/or three times a day (TID).
In another embodiment of the present disclosure the compound is administered in an amount of 0.01 mg/kg bodyweight to 40 mg/ kg bodyweight, such as 0.01 mg/ kg bodyweight to 0.05 mg/ kg bodyweight, 0.05 to 0.1 mg/ kg bodyweight, 0.1 to 0.5 mg/ kg bodyweight, 0.5 mg to 1 mg/ kg bodyweight, 1 to 2 mg/ kg bodyweight, 2 to 3 mg/ kg bodyweight, 3 to 5 mg/ kg bodyweight, 5 to 10 mg/ kg bodyweight, 10 to 15 mg/ kg bodyweight, 15 to 20 mg/ kg bodyweight, 20 to 30 mg/ kg bodyweight, for example 30 to 40 mg/ kg bodyweight.

Formulation

In one embodiment of the present disclosure the composition comprising a compound of formula (I), (Ia) or (II) as defined herein is a pharmaceutical composition, such as a pharmaceutically safe composition. The composition comprising a compound of formula (I), (Ia) or (II)) as defined herein may be administered in any suitable way e.g. orally, sublingually, or parenterally, and it may be presented in any suitable form for such administration, e.g. in the form of solutions, suspension, aerosols, tablets, capsules, powders, syrups, implant or dispersions for injection.
In one embodiment of the present disclosure, the composition comprising a compound of formula (I), (Ia) or (II) as defined herein is formulated as a suspension.

In one embodiment of the present disclosure, the composition comprising a compound of formula (I), (Ia) or (II) as defined herein is formulated as an oral dose form, such as a solid oral dose form or pharmaceutical entity, such as tablets or capsules, or a liquid oral dose form. Methods for the preparation of solid pharmaceutical preparations are well known in the art.

In another embodiment of the present disclosure the composition comprising a compound of formula (I), (Ia) or (II) as defined herein is formulated as an injectable dose form.

In one embodiment of the present disclosure the composition comprising a compound of formula (I), (Ia) or (II) as defined herein is formulated in the form of a solid pharmaceutical entity, suitably as a tablet or a capsule

The compound (I), (Ia) or (II) as the free base or the salt thereof may be administered alone or in combination with pharmaceutically acceptable carriers or excipients, in either single or multiple doses. The pharmaceutical compositions may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, Pa., 1995.

References

Lindskog et al. 2010 "Melanocortin 1 Receptor Agonists Reduce Proteinuria". J Am Soc Nephrol 21: 1290-1298, 2010.

Qiao et al. 2016 "MC1R is dispensable for the proteinuria reducing and glomerular protective effect of melanocortin therapy". Nature Scientific Reports 6:27589

Montero-Melendez et al. 2015 "Biased Agonism as a Novel Strategy To Harness the Proresolving Properties of Melanocortin Receptors without Eliciting Melanogenic Effects". J Immunol 2015; 194:3381-3388.

**Examples**

*Methods:*

Passive Heymann Nephritis was introduced to Male Sprague-Dawley rats by administration of anti-Fx1A serum (PTX-002S, Probetex, San Antonio, TX, USA)

Probetex, lot 169-6H) on study day 0 (1 ml serum) and on study day 7 (0.25 mL serum) into the jugular vein by slow injection during isoflurane anaesthesia. This dose regiment has previously (Lindskog et al, J Am Soc Nephrol. 2010; 21:1290-1298) been shown to induce significant proteinuria. Pharmacological intervention with control compound or test compound was initiated on day 14.

In one experiment (*experiment 1*) test compound 1 dissolved in isotonic saline was given once daily by intraperitoneal (ip) injections at a dose of 10 mg/kg (n=12) with animals treated with isotonic saline injections as controls (n=12).

In another experiment (*experiment 2*) test compound 1 was given once daily by oral gavage. Test compound 1 was given as a suspension in 10% hydroxypropyl-betacyclodextrin in water. Two doses of test compound 1 were tested: 20 mg/kg/day (n=8) and 40 mg/kg/day (n=8). In this experiment treatment effect of test compound 1 was compared to the effect of ACTH(1-24) as ACTH from the literature has been described to have treatment effects on proteinuria in the applied experimental model. For the positive control the rats (n=6) received ACTH(1-24) (Bachem cat no H-1150) treatment given at a dose of 10 μg/kg day by continuous subcutaneous infusion by use of Alzet osmotic mini-pumps for 4 weeks. This treatment regimen has previously been shown to induce around 50% reduction in proteinuria compared to untreated controls (Lindskog et al, J Am Soc Nephrol. 2010; 21:1290-1298).

In experiment 1 twenty-four hours urine collection were performed at three occasions, at day 14 following induction of nephritis, i.e. at the time point where pharmacological intervention was initiated, at day 28 and at day 42 following induction of nephritis. For urine collection the rats were placed in metabolic cages followed by a blood sample for measurement of plasma concentrations of creatinine and albumin. Likewise, the urine concentration of creatinine and albumin were determined and so was the volume of the 24-hours urine production.

In experiment 2 twenty-four hours urine collections was conducted at day 42 following induction if nephritis, i.e. following 28 days treatment with test compound 1 or ACTH(1-24). As in experiment 1 the rats were placed in metabolic cages followed by a blood sample for measurement of plasma concentrations of creatinine and albumin. Likewise, the urine concentration of creatinine and albumin were determined and so was the volume of the 24-hours urine production.

The blood sample was taken from the abdominal vein during pentobarbital (0.2-0.3 mL/rat ip; Dolethal, Vetoquinol via Centravet, Lapalisse, France). The collected blood was transferred into tubes coated with EDTA and centrifuged (10 minutes, 1000 g, 4°C) for plasma collection. Plasma samples were stored at -80°C until quantification of creatinine

and Albumin.

Albumin and creatinine in urine and plasma were quantified using a ABX Pentra 400 Clinical Chemistry analyzer (HORIBA).

*Albumin excretion rate* was determined as follows:

24 hours urine production x concentration of Albumin in urine.

*Creatinine clearance* as estimate of glomerular filtration rate was determined as follows:

(Urine concentration of creatinine x 24 hours urine production)/plasma concentration of creatinine

*Fractional Albumin excretion* was determined as follows:

((Urine concentration of urine x 24 hours urine production)/plasma concentration of albumin)/Creatinine Clearance x100

All data presented as mean ± Standard error of mean (SE). Statistical analyses were made by standard unpaired t-test, with p<0.05 considered statistical significant

*Results:*

Test compound 1 = AP1189 = (*(E)-N-trans*-{3-[1-(2-nitrophenyl)-1H-pyrrol-2-yl]-allylidene}-aminoguanidium acetate.

ACTH(1-24) = Adrenocorticotropic Hormone Fragment 1-24, human; Tetracosactide. Potent ACTH/MC2R agonist (EC50 = 5.5 nM). Stimulates glucocorticoid release from adrenal glands. Sequence: Ser-Tyr-Ser-Met-Glu-His-Phe-Arg-Trp-Gly-Lys-Pro-Val-Gly-Lys-Lys-Arg-Arg-Pro-Val-Lys-Val-Tyr-Pro.

Experiment 1:

Compared to control rats, rats treated with test compound 1 have a significant reduction in proteinuria at day 28 and day 42. At day 28 rats treated with test compound 1 had 31±8% reduction in albumin excretion rate and at day 42 a statistically significant 48±10% reduction in albumin excretion rate. In the control rats the albumin excretion rate was unchanged throughout (at day 28 96±11 % of day 14 and at day 42 102±14% of day 14) (See Fig. 1).

A comparable picture was identified when the fractional album excretion was evaluated: at day 28 FEAlb was reduced by 48±5% and at day 42 by 45±11 %. (No figure)

Experiment 2:

The animals treated with test compound 1 had a significantly higher GFR (creatinine clearance) following 4 weeks treatment than the rats treated with the positive control (ACTH 1-24 at 10 mg/day): GFR in rats treated with positive control: 2.19 ±0.24 mL/min compared to test compound 1 at 20 mg/kg: 3.12±0.14 mL/min (p=0.002 vs positive control) and test compound 1 at 40 mg/kg: 2.94±0.14 mL/24 min (p=0.01 vs positive control) (See Fig. 2A).

Treatment with test compound 1 significantly reduced the fraction albumin excretion (FEAlb) when compared to the positive control (ACTH 1-24 at 10 mg/day): FEAlb in positive control treated rats: 0.59±0.02% vs test compound 1 at 20 mg/kg: 0.045±0.04% (p=0.007 vs positive control) and test compound 1 at 40 mg/kg: 0.35±0.05% (p=0.001 vs positive control) (See Fig. 2B).

**Claims**

1. A composition comprising a compound of formula (Ia):

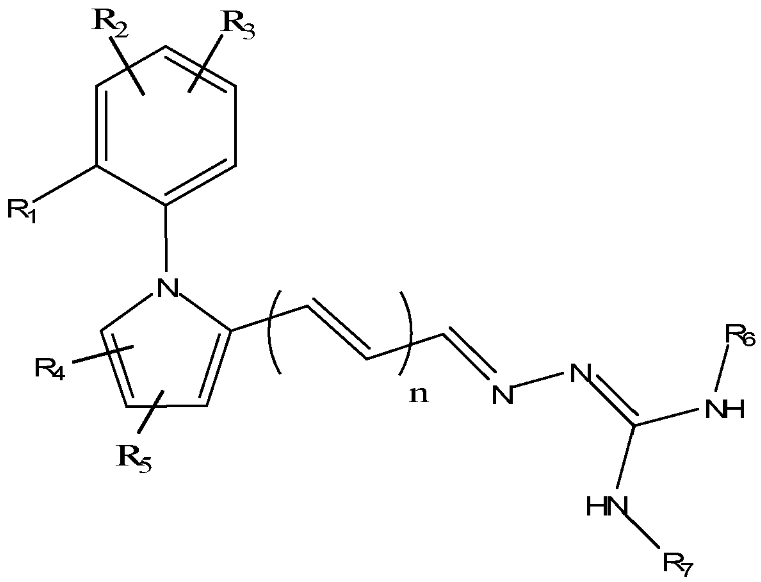

formula (Ia)

including tautomeric and stereoisomeric forms thereof; wherein n is 1; and

$R_1$ is $CF_3$, $CCl_3$, F, Cl, $NO_2$ or CN, and $R_2$, $R_3$, $R_4$, $R_5$, $R_6$, and $R_7$ are hydrogen; or a pharmaceutically acceptable salt thereof,
for use in the treatment of primary nephrotic syndrome (primary glomerulonephrosis).

2. The composition for use according to claim 1, wherein said compound is a biased agonist of the MC1R and MC3R, that does not stimulate melanogenesis via the MC1R.

3. The composition for use according to claim 1, wherein said primary nephrotic syndrome is membranous glomerulonephritis (MGN) (or membranous nephropathy (MN)).

4. The composition for use according to claim 1, wherein said primary nephrotic syndrome is focal segmental glomerulosclerosis (FSGS).

5. The composition for use according to claim 1, wherein said primary nephrotic syndrome is membranoproliferative glomerulonephritis (MPGN) (mesangiocapillary glomerulonephritis).; such as MPGN selected from Type 1 MPGN and Type 2 MPGN

6. The composition for use according to claim 1, wherein said primary nephrotic syndrome is rapidly progressive glomerulonephritis (RPGN) (crescentic GN).

7. The composition for use according to claim 1, wherein said primary nephrotic syndrome is minimal change disease (MCD).

8. The composition for use according to any of the preceding claims, wherein the compound is of formula (II)

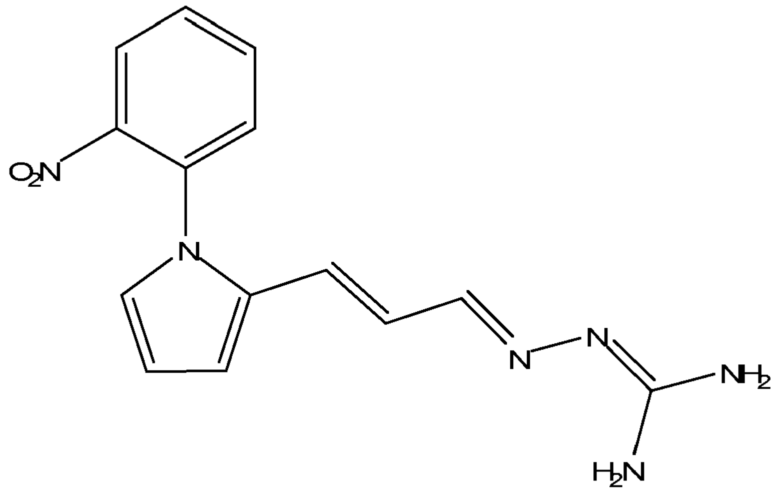

formula (II)

including tautomeric and stereoisomeric forms thereof; or a pharmaceutically acceptable salt thereof.

**9.** The composition for use according to any of the preceding claims, wherein said compound is selected from the group consisting of {3-[1-(2-nitrophenyl)-1 H-pyrrol-2-yl]-allylidene}-aminoguanidine and (*E*)-*N*-*trans*-{3-[1-(2-nitrophenyl)-1H-pyrrol-2-yl]-allylidene}-aminoguanidine, or a pharmaceutically acceptable salt thereof.

**10.** The composition for use according to any of the preceding claims, wherein said pharmaceutically acceptable salt is a pharmaceutically acceptable salt of an inorganic acid or an organic acid.

**11.** The composition for use according to claim 10, wherein said organic acid is selected from the group consisting of: formic acid, acetic acid, trichloroacetic acid, trifluoroacetic acid, propionic acid, benzoic acid, cinnamic acid, citric acid, fumaric acid, glycolic acid, lactic acid, maleic acid, malic acid, malonic acid, mandelic acid, oxalic acid, picric acid, pyruvic acid, salicylic acid, succinic acid, methanesulfonic acid, ethanesulfonic acid, tartaric acid, ascorbic acid, pamoic acid, bismethylene salicylic acid, ethanedisulfonic acid, gluconic acid, citraconic acid, aspartic acid, stearic acid, palmitic acid, EDTA, glycolic acid, p-aminobenzoic acid, glutamic acid, benzenesulfonic acid and p-toluenesulfonic acid; such as wherein said organic acid is acetic acid, succinic acid, tartaric acid or propionic acid; such as wherein said organic acid is acetic acid; and/or wherein said inorganic acid is selected from the group consisting of: hydrochloric acid, hydrobromic acid, hydroiodic acid, phosphoric acid, sulphuric acid and nitric acid.

**12.** The composition for use according to any of the preceding claims, wherein said compound is (*E*)-*N*-*trans*-{3-[1-(2-nitrophenyl)-1H-pyrrol-2-yl]-allylidene}-aminoguanidinium acetate (AP1189).

**13.** The composition for use according to any of the preceding claims, wherein said composition comprises, separately or together, one or more additional active pharmaceutical ingredients, such as one or more additional active pharmaceutical ingredients used for the treatment of kidney disease.

**14.** The composition for use according to any of the preceding claims, wherein said compound is administered in an amount of 1 mg to 1000 mg per day, such as 1 to 5 mg, 5 to 10 mg, 10 to 15 mg, 15 to 20 mg, 20 to 30 mg, 30 to 60 mg, 60 to 80 mg, 80 to 100 mg, 100 to 130 mg, 130 to 160 mg, 160 to 200 mg, 200 to 240 mg, 240 to 280 mg, 280 to 320 mg, 320 to 360 mg, 360 to 400 mg, 400 to 440 mg, 440 to 500 mg, 500 to 560 mg, 560 to 620 mg, 620 to 680 mg, 680 to 740 mg, 740 to 800 mg, 800 to 860 mg, 860 to 920 mg, 920 to 980 mg, 980 to 1000 mg, for example 500 to 1000 mg per day.

**15.** A composition comprising (*E*)-*N*-trans-{3-[1-(2-nitrophenyl)-1H-pyrrol-2-yl]-allylidene}-aminoguanidinium acetate (AP1189) for use in the treatment of primary nephrotic syndrome, such as minimal change disease (MCD).

## Patentansprüche

**1.** Zusammensetzung, umfassend eine Verbindung der Formel (Ia) :

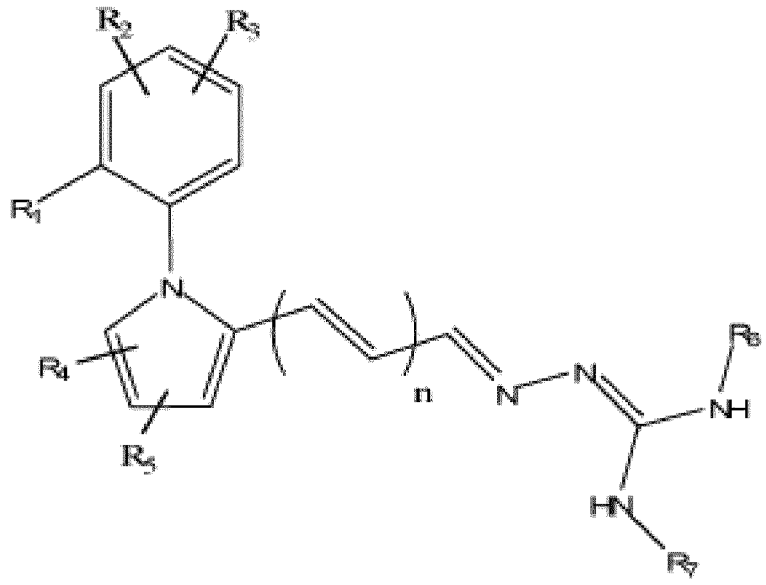

Formel (Ia)

beinhaltend tautomere und stereoisomere Formen davon;
wobei n 1 ist; und

$R_1$ $CF_3$, $CCl_3$, F, Cl, $NO_2$ oder CN ist, und $R_2$, $R_3$, $R_4$, $R_5$, $R_6$ und $R_7$ Wasserstoff sind;
oder ein pharmazeutisch akzeptables Salz davon,
zur Verwendung bei der Behandlung von primärem nephrotischen Syndrom (primäre Glomerulonephrose).

**2.** Zusammensetzung zur Verwendung nach Anspruch 1, wobei die Verbindung ein biased Agonist von dem MC1R und MC3R ist, der Melanogenese nicht über den MC1R stimuliert.

**3.** Zusammensetzung zur Verwendung nach Anspruch 1, wobei das primäre nephrotische Syndrom membranöse Glomerulonephritis (MGN) (oder membranöse Nephropathie (MN)) ist.

**4.** Zusammensetzung zur Verwendung nach Anspruch 1, wobei das primäre nephrotische Syndrom fokal segmentale Glomerulosklerose (FSGS) ist.

**5.** Zusammensetzung zur Verwendung nach Anspruch 1, wobei das primäre nephrotische Syndrom membranoproliferative Glomerulonephritis (MPGN) (mesangiokapilläre Glomerulonephritis) ist.; wie etwa MPGN ausgewählt aus MPGN Typ 1 und MPGN Typ 2

**6.** Zusammensetzung zur Verwendung nach Anspruch 1, wobei das primäre nephrotische Syndrom rasch progressive Glomerulonephritis (RPGN) (halbmondförmige-GN) ist.

**7.** Zusammensetzung zur Verwendung nach Anspruch 1, wobei das primäre nephrotische Syndrom Minimal Change Disease (MCD) ist.

**8.** Zusammensetzung zur Verwendung nach einem der vorhergehenden Ansprüche, wobei die Verbindung von Formel (II) ist

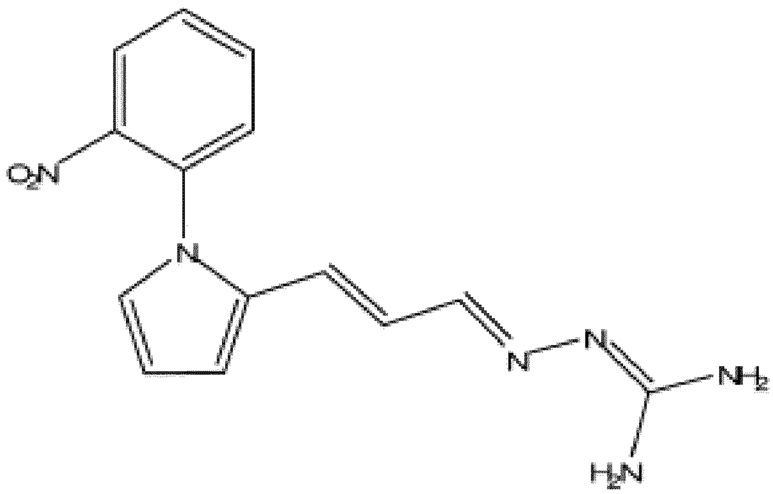

Formel (II)

beinhaltend tautomere und stereoisomere Formen davon; oder ein pharmazeutisch akzeptables Salz davon.

**9.** Zusammensetzung zur Verwendung nach einem der vorhergehenden Ansprüche, wobei die Verbindung aus der Gruppe bestehend aus {3-[1-(2-Nitrophenyl)-1H-pyrrol-2-yl]-allylidene}aminoguanidin und (*E*)-*N*-*trans*-{3-[1-(2-nitrophenyl)-1H-pyrrol-2-yl]-allylidene}-aminoguanidin oder einem pharmazeutisch akzeptablen Salz davon ausgewählt ist.

**10.** Zusammensetzung zur Verwendung nach einem der vorhergehenden Ansprüche, wobei das pharmazeutisch akzeptable Salz ein pharmazeutisch akzeptables Salz von einer anorganischen Säure oder einer organischen Säure ist.

**11.** Zusammensetzung zur Verwendung nach Anspruch 10, wobei die organische Säure aus der Gruppe bestehend aus Folgendem ausgewählt ist: Formylsäure, Essigsäure, Trichloressigsäure, Trifluoressigsäure, Propionsäure, Benzoesäure, Zimtsäure, Zitronensäure, Fumarsäure, Glycolsäure, Milchsäure, Maleinsäure, Apfelsäure, Malonsäure, Mandelsäure, Oxalsäure, Pikrinsäure, Pyruvinsäure, Salicylsäure, Succinsäure, Methansulfonsäure, Ethansulfonsäure, Weinsäure, Ascorbinsäure, Pamoasäure, Bismethylensalicylsäure, Ethandisulfonsäure, Gluconsäure, Citraconsäure, Asparaginsäure, Stearinsäure, Palmitinsäure, ETDA, Glycolsäure, p-Aminobenzoesäure, Glutaminsäure, Benzolsulfonsäure und p-Toluensulfonsäure; wie wobei die organische Säure Essigsäure, Succinsäure, Weinsäure oder Propioninsäure ist; wie wobei die organische Säure Essigsäure ist; und/oder wobei die anorganische Säure aus der Gruppe bestehend aus Folgendem ausgewählt wird: Hydrochlorsäure, Hydrobromsäure, Jodwasserstoffsäure, Phosphorsäure, Schwefelsäure und Salpetersäure.

**12.** Zusammensetzung zur Verwendung nach einem der vorhergehenden Ansprüche, wobei die Verbindung (*E*)-*N*-*trans*-{3-[1-(2-Nitrophenyl)-1H-pyrrol-2-yl]-allylidene}-aminoguanidin-acetat (AP1189) ist.

**13.** Zusammensetzung zur Verwendung nach einem der vorhergehenden Ansprüche, wobei die Verbindung einen oder mehrere zusätzliche aktive pharmazeutische Inhaltsstoffe, getrennt oder zusammen, umfasst, wie einen oder mehrere zusätzliche aktive pharmazeutische Inhaltsstoffe, die für die Behandlung von einer Nierenkrankheit verwendet werden.

**14.** Zusammensetzung zur Verwendung nach einem der vorhergehenden Ansprüche, wobei die Verbindung in einer Menge von 1 mg bis 1000 mg pro Tag verabreicht wird, wie 1 bis 5 mg, 5 bis 10 mg, 10 bis 15 mg, 15 bis 20 mg, 20 bis 30 mg, 30 bis 60 mg, 60 bis 80 mg, 80 bis 100 mg, 100 bis 130 mg, 130 bis 160 mg, 160 bis 200 mg, 200 bis 240 mg, 240 bis 280 mg, 280 bis 320 mg, 320 bis 360 mg, 360 bis 400 mg, 400 bis 440 mg, 440 bis 500 mg, 500 bis 560 mg, 560 bis 620 mg, 620 bis 680 mg, 680 bis 740 mg, 740 bis 800 mg, 800 bis 860 mg, 860 bis 920 mg, 920 bis 980 mg, 980 bis 1000 mg, zum Beispiel 500 bis 1000 mg pro Tag.

**15.** Zusammensetzung, umfassend (*E*)-*N*-*trans*-{3-[1-(2-Nitrophenyl)-1H-pyrrol-2-yl]-allylidene}-aminoguanidin-acetat (AP1189) zur Verwendung bei der Behandlung von primärem nephrotischen Syndrom, wie Minimal Change Disease (MCD).

## Revendications

**1.** Composition comprenant un composé de la formule (Ia) :

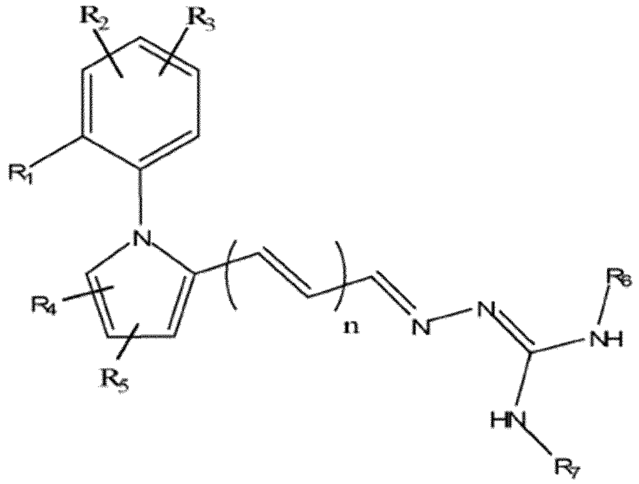

formule (Ia)

y compris les formes tautomères et stéréoisomères de celui-ci ;
dans laquelle n représente 1 ; et

$R_1$ représente $CF_3$, $CCl_3$, F, Cl, $NO_2$ ou CN, et $R_2$, $R_3$, $R_4$, $R_5$, $R_6$ et $R_7$ représentent un hydrogène ;
ou un sel pharmaceutiquement acceptable de celui-ci ;
à utiliser dans le traitement du syndrome néphrotique primitif (glomérulonéphrose primaire).

**2.** Composition à utiliser selon la revendication 1, dans laquelle ledit composé est un agoniste biaisé du MC1R et du MC3R, qui ne stimule pas la mélanogenèse par le biais du MC1R.

**3.** Composition à utiliser selon la revendication 1, dans laquelle ledit syndrome néphrotique primitif est la glomérulonéphrite extra-membraneuse (GEM) (ou la néphropathie extra-membraneuse (MN)).

**4.** Composition à utiliser selon la revendication 1, dans laquelle ledit syndrome néphrotique primitif est la glomérulosclérose segmentaire focale (FSGS).

**5.** Composition à utiliser selon la revendication 1, dans laquelle ledit syndrome néphrotique primitif est la glomérulonéphrite membranoproliférative (GNMP) (glomérulonéphrite mésangiocapillaire) ; telle que la GNMP choisie parmi la GNMP de type 1 et la GNMP de type 2.

**6.** Composition à utiliser selon la revendication 1, dans laquelle ledit syndrome néphrotique primitif est la glomérulonéphrite rapidement progressive (GNRP) (GN rapidement progressive).

**7.** Composition à utiliser selon la revendication 1, dans laquelle ledit syndrome néphrotique primitif est le syndrome néphrotique idiopathique (SNI).

**8.** Composition à utiliser selon l'une quelconque des revendications précédentes, dans laquelle le composé est de la formule (II)

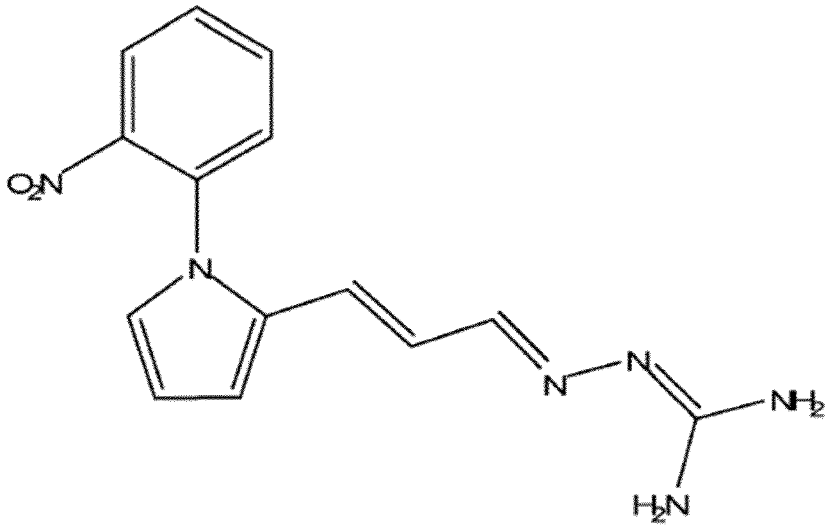

formule (II)

y compris les formes tautomères et stéréoisomères de celui-ci ; ou un sel pharmaceutiquement acceptable de celui-ci.

**9.** Composition à utiliser selon l'une quelconque des revendications précédentes, dans laquelle ledit composé est choisi dans le groupe constitué de {3-[1-(2-nitrophényl)-1H-pyrrol-2-yl]-allylidène}-aminoguanidine et de (*E*)-*N-trans*-{3-[1-(2-nitrophényl)-1H-pyrrol-2-yl]-allylidène}-aminoguanidine, ou d'un sel pharmaceutiquement acceptable de ceux-ci.

**10.** Composition à utiliser selon l'une quelconque des revendications précédentes, dans laquelle ledit sel pharmaceutiquement acceptable est un sel pharmaceutiquement acceptable d'un acide inorganique ou d'un acide organique.

**11.** Composition à utiliser selon la revendication 10, dans laquelle ledit acide organique est choisi dans le groupe constitué de : l'acide formique, l'acide acétique, l'acide trichloracétique, l'acide trifluoroacétique, l'acide propionique, l'acide benzoïque, l'acide cinnamique, l'acide citrique, l'acide fumarique, l'acide glycolique, l'acide lactique, l'acide maléique, l'acide malique, l'acide malonique, l'acide mandélique, l'acide oxalique, l'acide picrique, l'acide pyruvique, l'acide salicylique, l'acide succinique, l'acide méthanesulfonique, l'acide éthanesulfonique, l'acide tartrique, l'acide ascorbique, l'acide pamoïque, l'acide bisméthylènesalicylique, l'acide éthanedisulfonique, l'acide gluconique, l'acide citraconique, l'acide aspartique, l'acide stéarique, l'acide palmitique, l'EDTA, l'acide glycolique, l'acide p-aminobenzoïque, l'acide glutamique, l'acide benzène-sulfonique et l'acide p-toluènesulfonique ; dans laquelle, par exemple, ledit acide organique est l'acide acétique, l'acide succinique, l'acide tartrique ou l'acide propionique ; dans laquelle, par exemple, ledit acide organique est l'acide acétique ; et/ou dans laquelle ledit acide inorganique est choisi dans le groupe constitué de :

l'acide chlorhydrique, l'acide bromhydrique, l'acide iodhydrique, l'acide phosphorique, l'acide sulfurique et l'acide nitrique.

**12.** Composition à utiliser selon l'une quelconque des revendications précédentes, dans laquelle ledit composé est l'acétate de (*E*)-*N-trans*-{3-[1-(2-nitrophényl)-1H-pyrrol-2-yl]-allylidène}-aminoguanidinium (AP1189).

**13.** Composition à utiliser selon l'une quelconque des revendications précédentes, ladite composition comprenant, séparément ou ensemble, un ou plusieurs ingrédients pharmaceutiques actifs supplémentaires, tels qu'un ou plusieurs ingrédients pharmaceutiques actifs supplémentaires utilisés dans le traitement de la néphropathie.

**14.** Composition à utiliser selon l'une quelconque des revendications précédentes, dans laquelle ledit composé est administré dans une quantité de 1 mg à 1 000 mg par jour, telle que de 1 à 5 mg, de 5 à 10 mg, de 10 à 15 mg, de 15 à 20 mg, de 20 à 30 mg, de 30 à 60 mg, de 60 à 80 mg, de 80 à 100 mg, de 100 à 130 mg, de 130 à 160 mg, de 160 à 200 mg, de 200 à 240 mg, de 240 à 280 mg, de 280 à 320 mg, de 320 à 360 mg, de 360 à 400 mg, de 400 à 440 mg, de 440 à 500 mg, de 500 à 560 mg, de 560 à 620 mg, de 620 à 680 mg, de 680 à 740 mg, de 740 à 800 mg, de 800 à 860 mg, de 860 à 920 mg, de 920 à 980 mg, de 980 à 1 000 mg, par exemple de 500 à 1 000

mg par jour.

15. Composition comprenant de l'acétate de (*E*)-*N*-*trans*-{3-[1-(2-nitrophényl)-1H-pyrrol-2-yl-allylidène}-aminoguanidinium (AP1189) à utiliser dans le traitement du syndrome néphrotique primitif, tel que le syndrome néphrotique idiopathique (SNI).

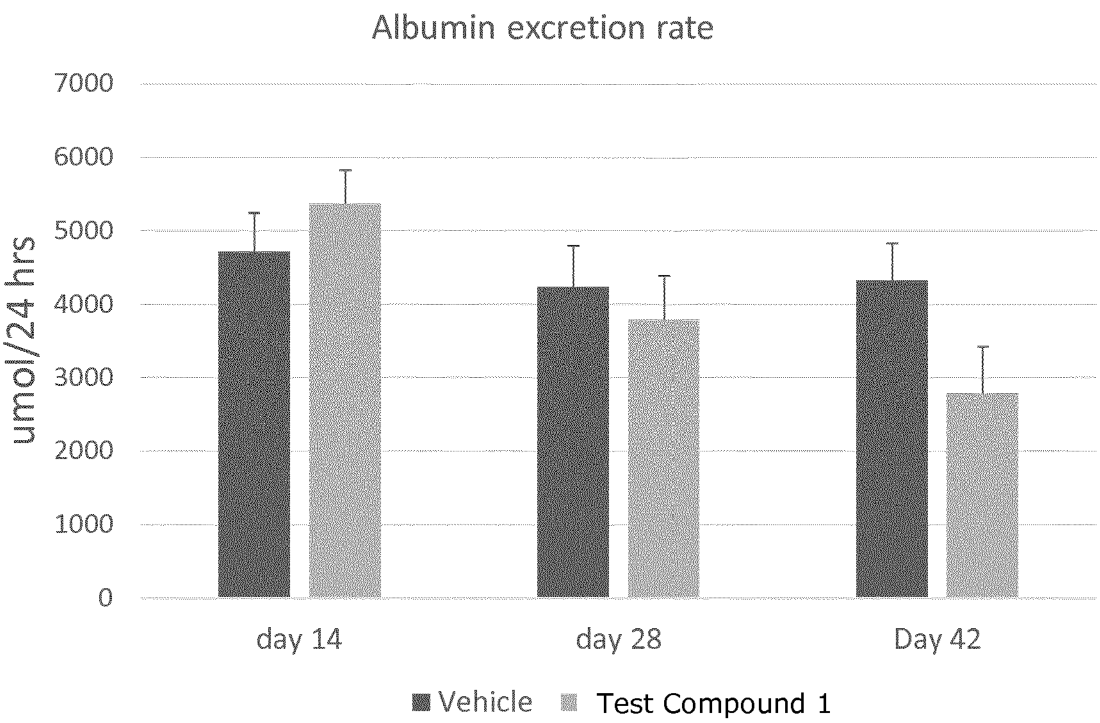
Albumin excretion rate
umol/24 hrs
7000
6000
5000
4000
3000
2000
1000
0
day 14
day 28
Day 42
Vehicle
Test Compound 1

Fig. 1

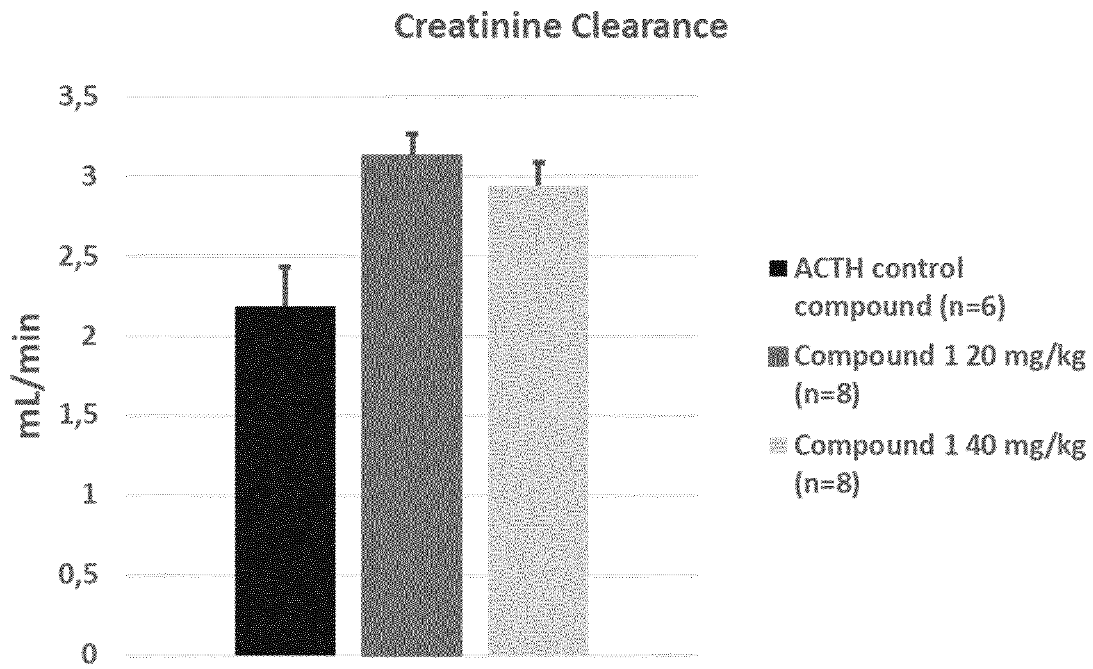
Creatinine Clearance
3,5
3
2,5
2
1,5
1
0,5
0
mL/min
ACTH control compound (n=6)
Compound 1 20 mg/kg (n=8)
Compound 1 40 mg/kg (n=8)

Fig. 2A

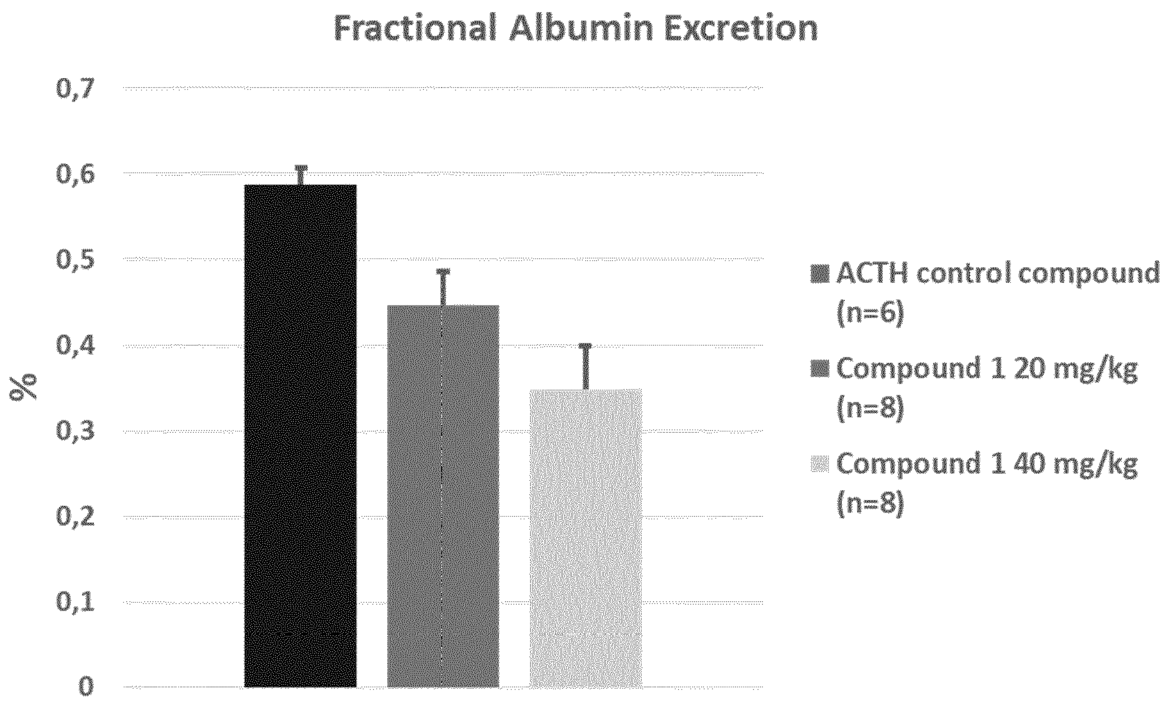
Fractional Albumin Excretion
0,7
0,6
0,5
0,4
0,3
0,2
0,1
0
%
ACTH control compound (n=6)
Compound 1 20 mg/kg (n=8)
Compound 1 40 mg/kg (n=8)

Fig. 2B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007141343 A


### Non-patent literature cited in the description


- *J. Pharm. Sci.,* 1977, vol. 66, 2
- *J. Org. Chem.,* 1958, vol. 23, 420
- Remington: The Science and Practice of Pharmacy. Mack Publishing Co, 1995
- **LINDSKOG et al.** Melanocortin 1 Receptor Agonists Reduce Proteinuria. *J Am Soc Nephrol,* 2010, vol. 21, 1290-1298
- **QIAO et al.** MC1R is dispensable for the proteinuria reducing and glomerular protective effect of melanocortin therapy. *Nature Scientific Reports,* 2016, vol. 6, 27589
- **MONTERO-MELENDEZ et al.** Biased Agonism as a Novel Strategy To Harness the Proresolving Properties of Melanocortin Receptors without Eliciting Melanogenic Effects. *J Immunol 2015,* 2015, vol. 194, 3381-3388
- **LINDSKOG et al.** *J Am Soc Nephrol.,* 2010, vol. 21, 1290-1298